(19) **Europäisches Patentamt European Patent Office Office européen des brevets**

(11) **EP 3 346 443 B1**

(12) **EUROPEAN PATENT SPECIFICATION**

(45) Date of publication and mention of the grant of the patent:
**27.10.2021 Bulletin 2021/43**

(51) Int Cl.:
**G01R 33/56** (2006.01)     **G01R 33/561** (2006.01)

(21) Application number: **16842129.5**

(22) Date of filing: **02.08.2016**

(86) International application number:
**PCT/KR2016/008494**

(87) International publication number:
**WO 2017/039163 (09.03.2017 Gazette 2017/10)**

(54) **METHOD FOR RESTORING MAGNETIC RESONANCE IMAGE AND MAGNETIC RESONANCE IMAGE PROCESSING APPARATUS**

VERFAHREN ZUR WIEDERHERSTELLUNG EINES MAGNETRESONANZBILDES UND MAGNETRESONANZBILDVERARBEITUNGSVORRICHTUNG

PROCÉDÉ DE RECONSTRUCTION D'IMAGE DE RÉSONANCE MAGNÉTIQUE ET APPAREIL DE TRAITEMENT D'IMAGE DE RÉSONANCE MAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.09.2015 RU 2015137794**
**11.07.2016 KR 20160087383**

(43) Date of publication of application:
**11.07.2018 Bulletin 2018/28**

(73) Proprietor: **Samsung Electronics Co., Ltd.**
**Suwon-si, Gyeonggi-do 16677 (KR)**

(72) Inventors:
- **KOROBCHENKO, Dmitry Alexandrovich**
  **Moscow 125581 (RU)**
- **MIGUKIN, Artem Sergeevich**
  **St.Petersburg 195112 (RU)**
- **DANILEVICH, Alexey Bronislavovich**
  **Moscow 125281 (RU)**
- **VARFOLOMEEVA, Anna Andreevna**
  **Moscow 127576 (RU)**
- **CHOI, Sang-cheon**
  **Suwon-si**
  **Gyeonggi-do 16484 (KR)**
- **SIROTENKO, Mikhail Yurievich**
  **Moscow 127224 (RU)**
- **RYCHAGOV, Michail Nikolaevich**
  **Moscow 124460 (RU)**

(74) Representative: **Gulde & Partner**
**Patent- und Rechtsanwaltskanzlei mbB**
**Wallstraße 58/59**
**10179 Berlin (DE)**

(56) References cited:
**WO-A1-2014/006633      KR-B1- 101 330 638**
**US-A1- 2004 165 757      US-A1- 2012 099 774**

- **BHAVE SAMPADA ET AL: "A variable splitting based algorithm for fast multi-coil blind compressed sensing MRI reconstruction", 2014 36TH ANNUAL INTERNATIONAL CONFERENCE OF THE IEEE ENGINEERING IN MEDICINE AND BIOLOGY SOCIETY, IEEE, 26 August 2014 (2014-08-26), pages 2400-2403, XP032674416, DOI: 10.1109/EMBC.2014.6944105 [retrieved on 2014-11-02]**
- **BERMAN B.P. ET AL.: "Dictionary Learning for Compressive T2 Mapping with Non-Cartesian Trajectories and Parallel Imaging", PROCEEDINGS OF THE INTERNATIONAL SOCIETY FOR MAGNETIC RESONANCE IN MEDICINE, 23ND ANNUAL MEETING AND EXHIBITION, TORONTO, CANADA, 30 MAY - 5 JUNE 2015, vol. 23, 15 May 2015 (2015-05-15), page 2502, XP040668179,**

EP 3 346 443 B1

- **ZHU PEIFEI ET AL: "Sparse decomposition learning based dynamic MRI reconstruction", VISUAL COMMUNICATIONS AND IMAGE PROCESSING; 20-1-2004 - 20-1-2004; SAN JOSE,, vol. 9445, 944516, 14 February 2015 (2015-02-14), XP060045586, DOI: 10.1117/12.2180534 ISBN: 978-1-62841-730-2**
- **CABALLERO JOSE ET AL: "Dictionary Learning and Time Sparsity for Dynamic MR Data Reconstruction", IEEE TRANSACTIONS ON MEDICAL IMAGING, IEEE SERVICE CENTER, PISCATAWAY, NJ, US, vol. 33, no. 4, 1 April 2014 (2014-04-01), pages 979-994, XP011544350, ISSN: 0278-0062, DOI: 10.1109/TMI.2014.2301271 [retrieved on 2014-03-31]**
- **GONG E. AND J.M. PAULY: "MRI Reconstruction by Learning the Dictionary of Spatial frequency-Bands Correlation: A novel algorithm integratable with PI and CS to further push acceleration", PROCEEDINGS OF THE INTERNATIONAL SOCIETY FOR MAGNETIC RESONANCE IN MEDICINE, 22ND ANNUAL MEETING AND EXHIBITION, MILAN, ITALY, 10-16 MAY 2014, vol. 22, 25 April 2014 (2014-04-25), page 744, XP040661824,**
- **TAO ZHANG ET AL: "Coil compression for accelerated imaging with Cartesian sampling", MAGNETIC RESONANCE IN MEDICINE., vol. 69, no. 2, 9 April 2012 (2012-04-09), pages 571-582, XP055496948, US ISSN: 0740-3194, DOI: 10.1002/mrm.24267**
- **RAVISHANKAR, SAIPRASAD ET AL.: 'MR Image Reconstruction from Highly Undersampled k-Space Data by Dietionary Leaming' IEEE TRANSACTION ON MEDICAL IMAGING vol. 30, no. 5, May 2011, pages 1028 - 1041, XP011321144**

**Description**

TECHNICAL FIELD

The present invention relates to medical image processing, and more particularly, to methods and apparatuses for reconstructing a magnetic resonance (MR) image of an object from partially sampled k-space data acquired by a plurality of receiver coils.

BACKGROUND ART

Magnetic resonance imaging (MRI) is a non-invasive imaging technique widely used in diagnosis since it can visualize both anatomical structures and physiological functions.

MRI is based on the principle of nuclear magnetic resonance. An alternating magnetic field and a radio frequency (RF) signal are used to excite hydrogen atoms to obtain information about a proton density distribution in parts of a body. A signal response from the excited hydrogen atoms is received by a set of so-called "receiver coils". These receiver coils may be a combination of a radio antenna (coil), an RF receiver, an amplifier, a detector, and other elements for performing reception, amplification, synchronization, and digitization of signals. Currently, MR scanners may include several of the RF receiver coils described above.

A plurality of RF receiver coils do not acquire a medical image of an object to be visualized but acquire a spatial discrete Fourier spectrum thereof. Such a spectrum is commonly called a "k-space". If one RF receiver coil is used, one k-space is acquired. If a plurality of RF receiver coils are used, an MR scanner provides several k-spaces corresponding to different RF receiver coils which respectively acquire signals from the object according to their spatial positions and individual parameters. The k-spaces corresponding to the different receiver coils are hereafter referred to as a multi-coil k-space. The multi-coil k-space may be fully sampled or partially sampled (undersampled). The partial sampling means that a part of the multi-coil k-space is skipped during acquisition. The reason for partial sampling is that full sampling requires a long scan time, and skipping a considerable part of the multi-coil k-space to be scanned may significantly reduce the medical image acquisition time.

However, examples of an MR image reconstructed from an undersampled k-space typically are of poor quality and are corrupted by strong aliasing artifacts. Thus, to improve the quality of reconstruction of an object, various post-processing procedures are applied. According to several authors the sensitivity of RF receiver coils is estimated to reconstruct the object directly from a plurality of aliased measurement values. Another approach is to directly recalculate skipped spectral elements of a multi-coil k-space by using a functional relationship between coils, typically called a "coil sensitivity map". The most representative methods among these types of approaches are Simultaneous Acquisition of Spatial Harmonics (SMASH) and Generalized Autocalibrating Partially Parallel Acquisitions (GRAPPA). Various versions of GRAPPA applied for k-space processing are described in patent documents US 8222900 B2 and US 2014/0218026 A1. In US 8427156 B2 and US 7884604 B2, a coil sensitivity map was used implicitly. However, accurately estimating the coil sensitivity map may be very difficult and time-consuming. An augmented Lagrangian-based method that is another known technology (for example, see WO 2013/067546 A1) is difficult to handle and is computationally complex. Thus, using the above-mentioned methods may be not actually sufficiently reasonable.

Furthermore, competing known MRI reconstruction techniques based on sparse approximation are used. One of these techniques is compressed sensing (CS), and the main idea of the CS is that real-time signals/images generally exhibit sparsity in a certain domain and can be accurately reconstructed (with acceptable quality) using a significantly smaller number of measurement values than that of unknowns. The main point is to find a proper sparse representation of an object in an overcomplete basis. An innovative CS technique initiated by Donoho (Patent Application US 2006/0029279) demonstrates that a used k-space does not need to be fully sampled for accurate reconstruction of an MR image. The CS exploits sparsity of an object in all measurements and has nowadays become a cutting-edge technology displaying great potential. However, since too many variational formulations of the optimization problem are generally used, it is necessary to specify a specific sparsifying transform. There are a lot of signal/image reconstruction techniques based on sparse object estimation, which are distinguished by a basis and sparsifying transform used. In particular, the object estimation is performed via a wavelet-like basis in the patent application publication (WO 2002/031756 A1), by using image characteristics called "similarity clusters" in the patent (US 8699773 B2), and by using a sparsified dataset produced from a previously acquired fully sampled image in the patent application (US 2010/0239143 A1). The above-mentioned patents do not focus on peculiarities of MR images, and the approaches described therein are not adapted to MR image characteristics, in contrast to other known dictionary-based sparse estimation methods.

According to generalized sensitivity encoding (GSENSE) and related applications, joint sparsity utilizes redundant information about an object. However, to significantly improve the reconstruction quality, a modern CS technique is used (see [R. Otazo et al, "Combination of compressed sensing and parallel imaging for highly accelerated first-pass cardiac perfusion MRI," Magn Reson Med. 64, 767-776 (2010)]). Patent US 8587307 B2 describes that CS is combined with parallel MRI (pMRI) and SENSE to reconstruct a resulting image from original folded images. The patent application (US 2013/0099786 A1) describes that the joint sparsity is used by a pMRI method such as the SENSE or pMRI with Adaptive Radius in k-Space (PARS) algorithm.

A crucial point of all CS algorithms lies in initialization of a measured undersampled multi-coil k-space. Most patent documents present results of application of default zero-filling. A number of patent documents (see, for example, WO 2014/075005 A1, WO 2012/144957 A1, and CN 103505207 A) propose MRI reconstruction using an iterative Split Bregman algorithm, but Split Bregman is never used in the GSENSE-like scenario for initialization. Due to this, it is possible to exploit the advantage of joint CS and fill unsampled positions in the measured multi-coil k-space with more accurate and proper values. The initialization based on a joint version of the Split Bregman algorithm is another key point of the proposed CS MRI reconstruction algorithms.

With advancements in parallel imaging, MRI data acquisition has been significantly accelerated due to redundant information about an object from arrays of receiver coils. Furthermore, MRI using receiver coil arrays may provide a higher signal-to-noise ratio than MRI using a single coil. At the same time, since an increase in the number of elements results in more datasets, image reconstruction requires more computation especially in the case of a 3D k-space. Coil compression (CC) algorithms (see, for example, WO 2012/123921 and [T. Zhang et al, "Coil compression for accelerated imaging with Cartesian sampling," Magn Reson Med. 69, 571-582 (2013)]) are effective in mitigating this problem by compressing data from multiple RF receiver coils into fewer so-called virtual receiver coils. CC linearly combines raw data without a change in sampling of spectra, such that a k-space from a virtual receiver coil is represented as a weighted sum of k-spaces obtained from RF receiver coils. A method of calculating weights is beyond the scope of the present application. The CC method has the advantage that it permits k-space processing for multiple receiver coils.

Moreover, the publication "A variable splitting based algorithm for Fast Multi-Coil Blind Compressed Sensing MRI reconstruction" (Bhave et al.) proposes a novel optimization algorithm that utilizes variable splitting strategies to significantly improve the convergence speed of the BCS optimization.

The publication "Dictionary Learning for Compressive T2 Mapping with Non-Cartesian Trajectories and Parallel Imaging" (Berman et al.) proposes a CS method that uses learned dictionaries to reconstruct images from multi-channel, non-Cartesian MRI data.

The publication "Sparse Decomposition Learning Based Dynamic MRI Reconstruction" (Zhu et al.) proposes to exploit a sparsity by proposed Sparse Decomposition Learning (SDL) algorithm, which is a combination of low-rank plus sparsity and Blind Compressed Sensing (BCS). The publication "Dictionary Learning and Time Sparsity for Dynamic MR Data Reconstruction" (Caballero et al.) proposes an iterative algorithm that enables the application of DL for the reconstruction of cardiac cine data with Cartesian undersampling by local processing of spatio-temporal 3D patches and by independent treatment of the real and imaginary parts of the dataset.

The publication "MRI Reconstruction by Learning the Dictionary of Spatialfrequency-Bands Correlation: A novel algorithm integratable with PI and CS to further push acceleration" (Gong et al.) proposes a new algorithm using Dictionary Learning to further improve reconstruction of MRI by exploiting the correlation between image details in different spatial-frequency bands.

In the present invention, a K-Singular Value Decomposition (K-SVD) algorithm (see patents WO2006/106508 A2 and US 2012/0177128 and [M. Aharon, M. Elad, and A. Bruckstein, "K-SVD: An Algorithm for Designing Overcomplete Dictionaries for Sparse Representation," IEEE Trans. Image Process. 54, 4311-4322 (2006)]) is used to learn a dictionary for CS image reconstruction. The dictionary is learned in advance of a sequence of fully-sampled MR images. A procedure of learning a dictionary departs from the scope of the present application.

Based on all the methods considered above, the patent application US 2013/0099786 A1 is found to be the closest to the proposed joint multi-dictionary CS MRI reconstruction method.

DETAILED DESCRIPTION OF THE INVENTION

TECHNICAL PROBLEM

The present invention and embodiments thereof relate to a method and apparatus according to the invention as defined in the appended claims for reconstructing a magnetic resonance (MR) image from a partially sampled (undersampled) multi-coil k-space. Since the total amount of time required to acquire data depends on the number of samples, a simple way to accelerate an MR data acquisition procedure is to reduce the amount of sampled frequencies and reconstruct an MR image that forms undersampled k-space spectra.

A method according to the present invention proposes combination of advantages of both parallel magnetic resonance imaging (pMRI) and a compressed sensing (CS) technique. However, unlike conventional methods, the method proposes use of the latest development of state-of-the-art CS MRI reconstruction based on a pre-learned dictionary using non-uniform sized patches. Dictionary-based sparse approximation applied to a single merged image may alternate with reconstruction of measured samples in the multi-coil k-space. Furthermore, several precomputed dictionaries targeted on various delicate features of different frequencies may be applied for the sparse approximation.

Undersampling of a multi-coil k-space may be performed by an MRI scanner according to a predefined sampling scheme (typically called a "sampling mask") represented by a binary matrix. "1" in the sampling mask may mean that a corresponding spatial frequency (a point on a k-space spectrum at a corresponding position) is sampled by a MR scanner, and "0" may mean that a respective frequency is not sampled. The result of sampling in accordance with the above-described scheme is an undersampled k-space. Undersampled values in an acquired k-space may be filled with some initial values, typically with zeros (0's). The sampling mask is the same for all RF receiver coils. The method takes as input a multi-coil k-space that is a plurality of identically undersampled k-space spectra obtained from different receiver coils. As the amount of sampled frequencies decreases, an undersampling rate of the multi-coil k-space may increase.

According to the embodiments, an object of the invention is to provide an MR image of an object with acceptable quality by performing the disclosed processing of an undersampled multi-coil k-space acquired from an MR scanner.

In the embodiments of the present invention, a CS technique is used for MR image reconstruction, and in particular, for sparse object approximation based on various precomputed dictionaries with non-uniform sized patches. There are several essential features in the embodiments of the present invention. Combining dictionary-based CS sparse approximation with joint sparsity of pMRI enables effective data aggregation in different receiver coils. This allows suppression of measurement noise, relatively fast reconstruction, and filtering of strong aliasing artifacts. Furthermore, dictionary-based CS is more flexible than fixed basis functions (e.g., wavelets) since it is adapted to MRI specifics through a dictionary learning process so there is no need to select an appropriate sparsifying transform. The efficiency of data initialization by the proposed novel joint Split Bregman and sparse object approximation based on multiple dictionaries may cause an increase in the convergence rate and allows reconstruction of details of an image even in the case of a high undersampling rate. Multiband decomposition used for processing of individual frequency bands in a multi-coil k-space may further improve an image reconstruction quality due to great sparsity within a frequency band.

TECHNICAL SOLUTION

As a technical solution to achieving the above technical problems, according to a first embodiment of the present invention, there is provided a magnetic resonance (MR) image processing apparatus as defined in independent claim 1, with preferred embodiments defined in dependent claims 2-10.

The second dictionary is acquired based on a residual between a pre-acquired image of the object and an approximation of the image based on the first dictionary.

The approximation may be a sparse approximation.

The processor may acquire a first image based on the partially sampled multi-coil k-space and obtain the reconstructed image by reconstructing the multi-coil k-space based on non-uniform sized patches of the first image, the first dictionary, and the second dictionary.

The processor may obtain the reconstructed image by reconstructing the multi-coil k-space based on patches corresponding to different frequency bands, which are acquired based on the partially sampled multi-coil k-space, the first dictionary, and the second dictionary.

The MR image processing apparatus may further include an operator console, and the operator console may include a display configured to visualize the reconstructed image.

The operator console may further include a controller, and the controller may be configured to control the MR image processing apparatus.

The MR image processing apparatus may further include an MR scanner configured to acquire data of the partially sampled multi-coil k-space.

According to an embodiment, the processor may decompose the acquired partially sampled multi-coil k-space into a plurality of frequency bands and obtain the reconstructed image by respectively reconstructing a plurality of partially sampled multi-coil k-spaces corresponding to the plurality of frequency bands based on the first and second dictionaries.

The processor may preprocess the partially sampled multi-coil k-space and reconstruct the preprocessed multi-coil k-space based on the first and second dictionaries.

The processor may preprocess the partially sampled multi-coil k-space by filling unsampled positions in the partially sampled multi-coil k-space with initial guesses and performing coil compression on the partially sampled multi-coil k-space.

According to a second embodiment of the present invention, there is provided a method of reconstructing an MR image as defined in the appended claims.

Furthermore, preprocessing of the partially sampled multi-coil k-space may include filling unsampled positions in the partially sampled multi-coil k-space with initial guesses and performing coil compression on the partially sampled multi-coil k-space.

Furthermore, the filling of the unsampled positions in the multi-coil k-space with the initial guesses may include filling the unsampled positions with at least one of a constant, random complex values, and values obtained by a joint Split Bregman iterative algorithm.

Furthermore, reconstructing the preprocessed multi-coil k-space by using multiband joint compressed sensing (CS) reconstruction may include: acquiring a plurality of multi-coil k-spaces corresponding to different frequency bands by decomposing the multi-coil k-space into multiple bands; acquiring coil sensitivity maps based on the plurality of multi-coil k-spaces corresponding to the different frequency bands; performing multi-dictionary joint CS reconstruction of the plurality of multi-coil k-spaces corresponding to the different frequency bands; acquiring a final multi-coil k-space based on the reconstructed plurality of multi-coil k-spaces; and obtaining an MR image from the acquired final multi-coil k-space.

Furthermore, the performing of the multi-dictionary joint CS reconstruction of the plurality of multi-coil k-spaces corresponding to the different frequency bands may include: acquiring a merged image from the plurality of multi-coil k-spaces corresponding to the different frequency bands; acquiring an approximation of the merged image from the merged image based on multi-dictionary sparse approximation; calculating multi-coil k-space data from the approximation of the merged image; and reconstructing the calculated multi-coil k-space data.

Furthermore, the acquiring of the merged image from the plurality of multi-coil k-spaces may include acquiring a plurality of coil images by performing a coil-wise inverse Fourier transform on the plurality of multi-coil k-spaces and projecting the plurality of coil images respectively onto spatial positions on the coil sensitivity maps.

The acquiring of the approximation of the merged image from the merged image may include: acquiring a sparse code by performing patch-based multi-dictionary sparse coding; and acquiring an approximation of the merged image by approximating the merged image based on the acquired sparse code.

Furthermore, the calculating of the multi-coil k-space data from the approximation of the merged image may include: rescaling the coil sensitivity maps; and decomposing the approximation of the merged image into a plurality of coil images and performing Fourier transform on the plurality of coil images.

Furthermore, the acquiring of the approximation of the merged image from the merged image based on the multi-dictionary sparse approximation may include: extracting overlapping patches from the merged image; performing dictionary-based sparse approximation of the extracted patches; and acquiring the approximation of the merged image by assembling the patches that have undergone the dictionary-based sparse approximation with the overlapping patches.

Furthermore, in the performing of the dictionary-based sparse approximation of the extracted patches, at least one of an original-oriented dictionary and a residual-oriented dictionary may be used.

## DESCRIPTION OF THE DRAWINGS

FIG. 1 illustrates a magnetic resonance (MR) image processing apparatus according to an example embodiment.

FIG. 2 is a flowchart of a joint compressed sensing (CS) magnetic resonance imaging (MRI) reconstruction method according to an example embodiment.

FIGS. 3A and 3B are flowcharts of joint CS MR imaging (MRI) reconstruction methods according to example embodiments.

FIG. 4 is a flowchart of data initialization based on a joint Split Bregman iterative algorithm according to an example embodiment.

FIG. 5 illustrates a dictionary-based joint CS reconstruction method according to an example embodiment.

FIG. 6 illustrates single-dictionary sparse approximation of patches according to an example embodiment.

FIG. 7 illustrates multi-dictionary sparse approximation of patches according to an example embodiment.

FIGS. 8A through 8C illustrate dictionary-based sparse approximations according to example embodiments.

FIG. 9 illustrates a process of learning original- and residual-oriented dictionaries.

FIG. 10 illustrates a joint CS MRI reconstruction system according to an example embodiment.

FIG. 11 illustrates a multi-dictionary CS MRI reconstruction system according to an example embodiment.

FIG. 12 illustrates a system for learning original- and residual-oriented dictionaries according to an example embodiment.

FIG. 13 illustrates an MR image processing system according to an example embodiment.

FIG. 14 illustrates a system for joint Split Bregman reconstruction according to an example embodiment.

FIG. 15 illustrates a user scenario 1 according to an example embodiment.

FIG. 16 illustrates a user scenario 2 according to an example embodiment.

FIG. 17 illustrates a user scenario 3 according to an example outside the scope of the present invention.

FIG. 18 is a schematic diagram of an MRI system.

## MODE OF THE INVENTION

Like reference numerals refer to like elements throughout. The present specification does not describe all components in the embodiments, and common knowledge in the art or the same descriptions of the embodiments will be omitted below. The term "part" or "portion" may be implemented using hardware or software, and according to embodiments, one "part" or "portion" may be formed as a single unit or element or include a plurality of units or elements. Hereinafter, the principles and embodiments of the present disclosure will be described in detail with reference to the accompanying drawings.)

In the present specification, an "image" is a medical image obtained by a magnetic resonance imaging (MRI) apparatus.

Furthermore, in the present specification, an "object" may be a target to be imaged and include a human, an animal, or a part of a human or animal. For example, the object may include a body part (an organ) or a phantom. )

An MRI system acquires an MR signal and reconstructs the acquired MR signal into an image. The MR signal denotes a radio frequency (RF) signal emitted from the object.

In the MRI system, a main magnet creates a static magnetic field to align a magnetic dipole moment of a specific atomic nucleus of the object placed in the static magnetic field along a direction of the static magnetic field. A gradient coil may generate a gradient magnetic field by applying a gradient signal to a static magnetic field and induce resonance frequencies differently according to each region of the object.

An RF coil may emit an RF signal to match a resonance frequency of a region of the object whose image is to be acquired. Furthermore, when gradient magnetic fields are applied, the RF coil may receive MR signals having different resonance frequencies emitted from a plurality of regions of the object. Though this process, the MRI system may obtain an image from an MR signal by using an image reconstruction technique.

To create an image of an object being scanned, a signal response from excited hydrogen atoms is received by a set of radio frequency (RF) receiver coils. An RF receiver coil may be a combination of a radio antenna, an RF receiver, an amplifier, a detector, and several other elements that perform reception, amplification, synchronization, and digitization of signals. The set of RF receiver coils are used to simultaneously obtain a response signal from an object. The RF receiver coils each have different spatial locations and parameter settings. Such parallel data collection may result in redundancy of information about the object during joint CS reconstruction. The acquisition time may be reduced by partial sampling or undersampling k-spaces. The undersampling of a k-space is performed by applying a predetermined sampling mask which is the same for all receiver coils. Values in unsampled positions may be filled with some initial values (typically with zeros). Such an undersampled multi-coil k-space is used as an input of CS MRI reconstruction algorithms according to an embodiment.

In embodiments of the present invention, there are no limitations in terms of the technical specification and configuration of a receiver coil antenna.

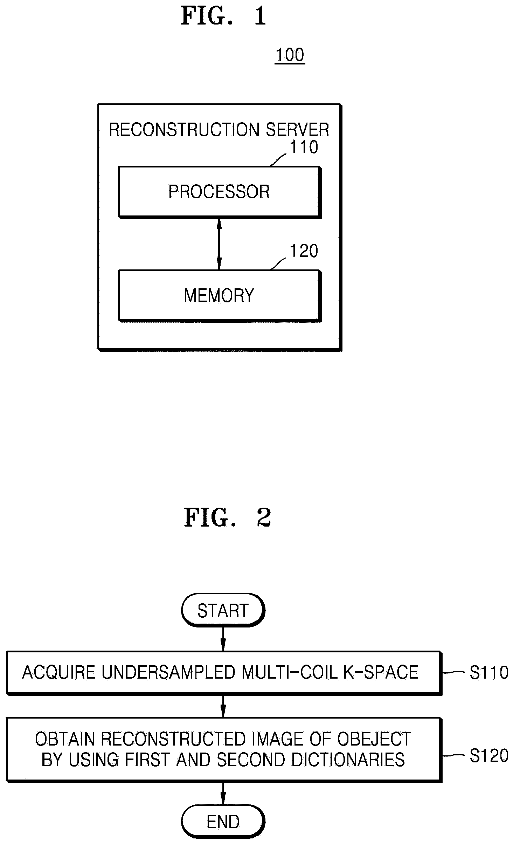

FIG. 1 illustrates an MRI apparatus for joint MRI reconstruction according to an example embodiment.

According to an embodiment, as shown in FIG. 1, the MRI apparatus may be implemented as a reconstruction server 100. The reconstruction server 100 may be an apparatus for processing MR signals acquired by performing an MRI scan on an object.

The reconstruction server 100 may receive MR signals from an MR scanner (not shown). Furthermore, the reconstruction server 100 may receive multi-coil k-space data corresponding to the MR signals from the MR scanner. The reconstruction server 100 may include a server apparatus for reconstructing an MR image from the multi-coil k-space data corresponding to the MR signals. In this case, the server apparatus may be a server apparatus in a hospital, which performs an MRI scan on a patient, or a medical server apparatus located outside the hospital.

The reconstruction server 100 may include a processor 110 and a memory 120. However, this is merely an example, and the reconstruction server 100 may include more components than those shown in FIG. 1.

The processor 110 may acquire an undersampled multi-coil k-space with respect to the object. According to an embodiment, the processor 110 may receive the undersampled multi-coil k-space from the MR scanner.

According to an embodiment, the processor 110 obtains a reconstructed image of the object by reconstructing the multi-coil k-space based on a plurality of dictionaries. A dictionary may mean data precomputed for simplifying calculations. For example, the dictionary may include a table, a look-up table, etc. In this case, the processor 110 may run an image reconstruction program stored in the memory 120. Furthermore, the processor 110 may include a module for reconstructing undersampled MRI data.

The processor 110 also acquires a reconstructed image of the object by reconstructing the multi-coil k-space based on a pre-acquired first dictionary and a second dictionary acquired using the first dictionary. In this case, the reconstructed image means an MR image of the object to be finally obtained. According to the invention, the second dictionary is acquired based on a residual between a pre-acquired image of the object and a sparse approximation of the pre-acquired image based on the first dictionary.

The processor 110 may also acquire a first image based on the undersampled multi-coil k-space and obtain a reconstructed image of the object by reconstructing the multi-coil k-space based on non-uniform sized patches of the first image and the first and second dictionaries. In this case, the first image may be an image obtained by merging images acquired from a plurality of multi-coil k-spaces corresponding to different frequency bands

Furthermore, the processor 110 may obtain a reconstructed image of the object by reconstructing the multi-coil k-space based on patches corresponding to different frequency bands, which are acquired based on the undersampled multi-coil k-space, and the first and second dictionaries.

Furthermore, the processor 110 may decompose the acquired undersampled multi-coil k-space into a plurality of frequency bands and obtain a reconstructed image by respectively reconstructing undersampled multi-coil k-spaces corresponding to the plurality of frequency bands based on the first and second dictionaries.

Furthermore, the processor 110 may preprocess the undersampled multi-coil k-space and reconstruct the preprocessed multi-coil k-space based on the first and second dictionaries.

Furthermore, the processor 110 may preprocess the undersampled multi-coil k-space by filling unsampled positions in the undersampled multi-coil k-space with initial guesses and performing coil compression on the undersampled multi-coil k-space.

For example, the initial guesses with which the processor 110 fills the unsampled positions may include a constant, random complex values, or values obtained by a joint Split Bregman iterative algorithm. For example, the initial guesses may be 0. For example, the coil compression performed by the processor 110 may include compressing data from multiple RF receiver coils into fewer virtual receiver coils. According to an embodiment, the processor 110 may reconstruct the preprocessed multi-coil k-space by using a reconstruction algorithm. For example, the processor 110 may reconstruct the preprocessed multi-coil k-space by using multiband joint compressed sensing (CS) reconstruction. A process of reconstructing the preprocessed multi-coil k-space by using the multiband joint CS reconstruction may include decomposing the undersampled multi-coil k-space into a plurality of frequency bands (e.g., low and high frequency bands).

The processor 110 may perform multi-dictionary joint CS reconstruction respectively on a plurality of multi-coil k-spaces corresponding to the plurality of frequency bands. Furthermore, the processor 110 may obtain a reconstructed MR image by merging the result of the reconstruction of the plurality of multi-coil k-spaces. However, a process, performed by the reconstruction server 100, of reconstructing an MR image by using the multi-band joint CS reconstruction is not limited thereto, and may include more steps than those described above.

According to an embodiment, the memory 120 may store a plurality of dictionaries acquired before acquiring the undersampled multi-coil k-space. According to the invention, the memory 120 stores a pre-acquired first dictionary and a second dictionary acquired using the first dictionary. Furthermore, the memory 120 stores a pulse sequence, coil sensitivity maps, etc., necessary for reconstructing an MR image.

The MRI apparatus may further include an operator console (not shown). The operator console may include a controller (not shown) configured to control an MR image processing system and a display (not shown) configured to display a reconstructed MR image.

Furthermore, the MRI apparatus may further include an MR scanner (not shown) containing at least one receiver coil configured to acquire a k-space.

FIG. 2 is a flowchart of a method of reconstructing an MR image according to an example embodiment.

Steps of the method of reconstructing an MR image may be performed by the MRI apparatus described with reference to FIG. 1

In step S110, the MRI apparatus obtains an undersampled multi-coil k-space with respect to an object (S110).

Furthermore, in step S120, the MRI apparatus obtains a reconstructed image of the object by using first and second dictionaries (S120). In detail, the MRI apparatus obtains a reconstructed image of the object by reconstructing the multi-coil k-space based on a pre-acquired first dictionary and a second dictionary acquired using the first dictionary.

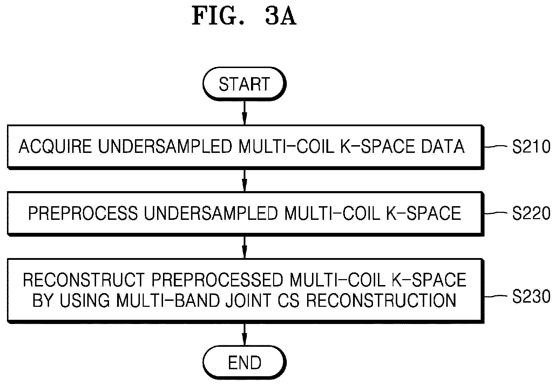

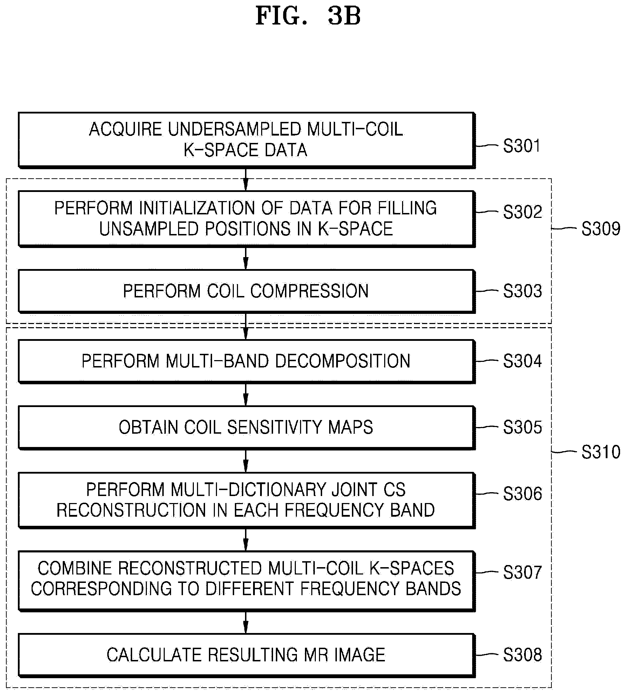

FIGS. 3A and 3B are flowcharts of joint CS MRI reconstruction methods according to example embodiments.

According to an embodiment, referring to FIG. 3A, the joint CS MRI reconstruction method may include three main steps. Furthermore, referring to FIG. 3B, the joint CS MRI reconstruction method may include detailed steps.

According to an embodiment, the joint CS MRI reconstruction method may include the step of acquiring an undersampled multi-coil k-space to be reconstructed (S210). The undersampled multi-coil k-space is received from an MR scanner (not shown). The undersampled multi-coil k-space is acquired according to a predetermined sampling scheme (sampling mask).

According to an embodiment, the joint CS MRI reconstruction method may include the step of preprocessing the acquired undersampled multi-coil k-space (S220 and S309). In steps S220 and S309 of the preprocessing, initialization of the multi-coil k-space may be performed in order to fill unsampled positions with some initial values (S302). Different types of initialization including zeros or random complex values may be applied. According to an embodiment, a joint Split Bregman iterative algorithm may be applied to this initialization procedure. According to an embodiment, the multi-coil k-space initialized in steps S220 and S309 may be compressed by performing coil compression (CC) to reduce the total number of k-spaces (S303). K-spaces generated by performing the CC may be related to so-called "virtual coils" which are used instead of RF receiver coils.

According to an embodiment, the joint CS MRI reconstruction method may include the step of reconstructing the preprocessed multi-coil k-space by using multi-band joint CS reconstruction (S230 and S310). The multi-coil k-space may be decomposed into several frequency bands in such a manner that the generated multi-coil k-spaces may correspond respectively to the frequency bands (S304). Furthermore, the coil sensitivity maps may be obtained based on a fully sampled part of k-space data (S305). Furthermore, each of the multi-coil k-spaces may be reconstructed using the multi-dictionary joint CS algorithm to restore unsampled frequencies within its corresponding frequency band (S306). Furthermore, all the multi-coil k-spaces corresponding to different frequency bands may be combined together to form a final multi-coil k-space (S307). In addition, a resulting MR image may be calculated from the final multi-coil k-space (S308).

The basics of the used CS technique and parallel MRI (pMRI) reconstruction are now described.

**SENSE-like iterative MRI reconstruction**

PMRI algorithms are inextricably connected to coil sensitivity maps used to combine measured k-spaces. A physical relation between a proton density $\pi$ of an object to be measured and its undersampled Fourier spectrum $u_i$ acquired via an i-th receiver coil may be expressed as shown in Equation (1):

$$\mathbf{u}_i = F_u(\mathbf{p}_i), \; \mathbf{p}_i(r) = \mathbf{s}_i(r) \cdot \boldsymbol{\pi}(r) \, , \qquad \ldots (1)$$

where $r$ is a spatial coordinate, $F_u$ is an undersampled Fourier transform, $s_i(r)$ is a value of a coil sensitivity map of an i-th receiver coil at the spatial coordinate r. Equation (1) explains how object $\pi$ produces a difference between multiple k-spaces due to parameter settings and positions of receiver coils.

When $\circ$ denotes an element-wise Hadamard product, Equation (1) may be rewritten in a vector form as follows:

$$\mathbf{u}_i = F_u(\mathbf{s}_i \circ \boldsymbol{\pi}), \; \forall i \qquad \ldots (2)$$

It is convenient to represent a property of interest of the object (proton density) as follows:

$$\hat{\boldsymbol{\pi}} = \sum_i \mathbf{s}_i^H F_u^{-1}(\mathbf{u}_i)/(\mathbf{s}_i^H \mathbf{s}_i) \, , \qquad \ldots (3)$$

where superscript $^H$ denotes the Hermitian conjugate and $F_u^{-1}$ is undersampled inverse Fourier transform. Complex-valued vectors of coil sensitivity maps and k-spaces are used herein A bold lower case letter denotes a column vector.

Equations (1) and (2) are used to describe splitting the object into the multi-coil k-space, In other words, photon density $\pi$ is split into several images $p_i$, corresponding to various receiver coils via individual coil sensitivity maps $s_i$, and then the images $p_i$, are transmitted to the Fourier domain. An image $p_i$, is commonly referred to as an i-th "coil image". Equation (3) defines an arithmetic operation that merges coil images obtained from corresponding k-spaces. The above-described arithmetic operations (splitting and merging) combine the proposed joint CS MRI reconstruction with generalized sensitivity encoding (GSENSE).

**Initialization by joint Split Bregman**

In a method according to an embodiment, the inventors propose a novel modification of a Split Bregman iterative algorithm for efficient data initialization. According to [T. Goldstein, and S Osher, "The Split Bregman method for l1-regularized problems," SIAM J. on Imag. Sciences 2, 323-343 (2009)], the joint CS reconstruction problem may be formulated for measurements obtained by various receiver coils as the following basis pursuit denoising problem.

$$\boldsymbol{\pi} = \arg\min_{\boldsymbol{\pi}} \sum_i \left\| F_u(\mathbf{s}_i \circ \boldsymbol{\pi}) - \mathbf{u}_i \right\|_2^2 + \lambda \cdot \left\| \Psi(\boldsymbol{\pi}) \right\|_1 \qquad \ldots (4)$$

This results in minimization of a convex criterion function. Here $\lambda$ is a regularizing parameter, $\Psi$ is a sparsifying transform (total variation) and the $\ell$1-norm $\|...\|^1$ is defined as the sum of absolute values of items of the vector. According to [Y. Wang, et at., "A fast algorithm for image deblurring with total variation regularization," CAAM Technical Report (2007)], $\ell_1$- and $\ell_2$-norms in Equation (4) are decoupled as follows:

$$\min_{\boldsymbol{\pi},\mathbf{d}} \sum_i \left\| F_u(\mathbf{s}_i \circ \boldsymbol{\pi}) - \mathbf{u}_i \right\|_2^2 + \lambda \cdot \left\| \mathbf{d} \right\|_1 + \mu \cdot \left\| \Psi(\boldsymbol{\pi}) - \mathbf{d} \right\|_2^2 \qquad \ldots (5)$$

where d is a sparse object approximation. It follows the joint Split Bregman algorithm below:

$$\boldsymbol{\pi}^{k+1} = \arg\min_{\boldsymbol{\pi}} \sum_i \left\| F_u(\mathbf{s}_i \circ \boldsymbol{\pi}) - \mathbf{u}_i \right\|_2^2 + \mu \cdot \left\| \mathbf{d}^k - \Psi(\boldsymbol{\pi}) - \mathbf{b}^k \right\|_2^2 \qquad \ldots (6.1)$$

$$\mathbf{d}^{k+1} = \arg\min_{\mathbf{d}} \lambda \cdot \left\| \mathbf{d} \right\|_1 + \mu \cdot \left\| \mathbf{d} - \Psi(\boldsymbol{\pi}^{k+1}) - \mathbf{b}^k \right\|_2^2 \qquad \ldots (6.2)$$

$$\mathbf{b}^{k+1} = \mathbf{b}^k + \Psi(\boldsymbol{\pi}^{k+1}) - \mathbf{d}^{k+1} \qquad \ldots (6.3)$$

where $b^k$ denotes a vector of auxiliary Bregman parameters at a k-th iteration. In contrast to a conventional Split Bregman algorithm with measurements for a single receiver coil, joint formulation shown in Equation (6.1) may have all advantages of pMRI algorithms.

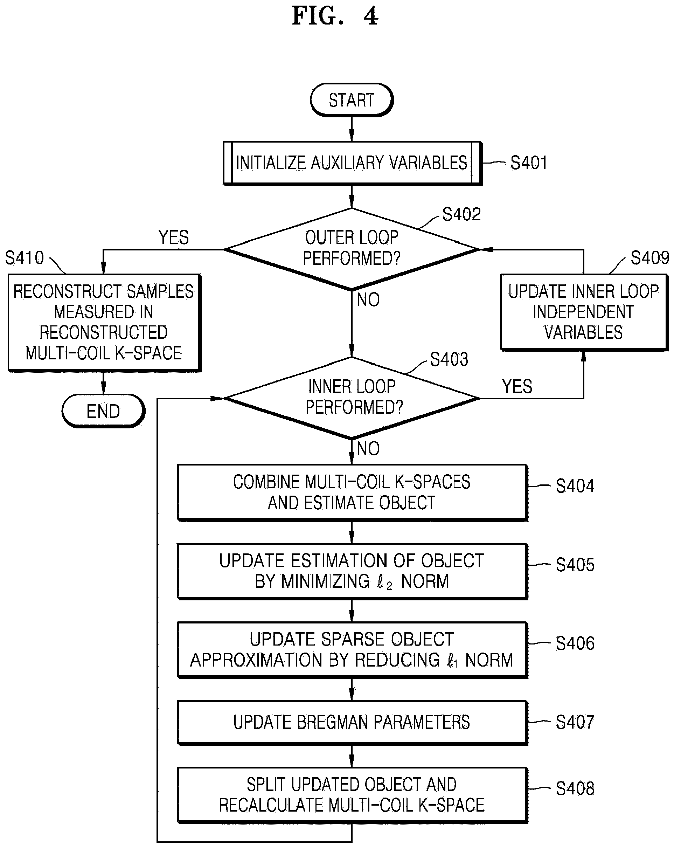

FIG. 4 is a flowchart of data initialization based on a joint Split Bregman iterative algorithm according to an example embodiment.

According to [T. Goldstein, and S. Osher, "The Split Bregman method for l1-regularized problems," SIAM J. on Imag. Sciences 2, 323-343 (2009)] and GSENSE, an implementation of the joint Split Bregman algorithm shown in Equations (6.1) through (6.3) may consist of two nested loops. In a first step (S401), auxiliary variables of sparse object approximation d° and Bregman parameter b° may be initialized with zero vectors. During an inner loop, object $\pi$, its sparse approximation d, and auxiliary parameter vector b may be recalculated. In step S404, object $\pi$ may be estimated according to Equation (3) by performing inverse Fourier transform of k-spaces from different receiver coils together with merging of the estimated images. Thereafter, object recalculation (S405) and updating of object approximation d (S406) may be performed by minimizing Equations (6.1) and (6.2), respectively. Optimization with respect to $\ell_1$, norm (S406) may be achieved via soft thresholding with a threshold equal to $1/2\mu$. In step S407, Bregman parameter vector b may be updated according to Equation (6.3). Finally, the resulting object may be represented by several k-spaces corresponding to the coil images in accordance with Equation (2). The inner loop is finished when stopping condition (S403) is met, and then inner-loop independent variables may be updated according to measured k-spaces corresponding to different receiver coils and reconstruction of the object (S409).

After conditions of an outer loop is satisfied (S402), a recovered object estimation may be updated by reconstructing k-space samples measured in a reconstructed Fourier spectrum (S410). This procedure ensures that the joint Split Bregman algorithm operates only over unsampled frequencies by preparing data for joint CS reconstruction without making a change to the given k-space measurements. If the arithmetic operation is finished, the initialization based on the joint Split Bregman algorithm n may be completed.

**Dictionary-based sparse object approximation**

The main part of the method according to the embodiment is based on sparse approximation of an input volumetric signal using a precomputed dictionary. At this step, the following optimization problem may be solved:

$$\boldsymbol{\pi} = \arg\min_{\boldsymbol{\pi}} \sum_i \left\| F_u(\mathbf{s}_i \circ \boldsymbol{\pi}) - \mathbf{u}_i \right\|_2^2 + \lambda \cdot \left\| \Psi(\boldsymbol{\pi}) \right\|_0 \qquad \ldots (7)$$

Instead of the $\ell_1$-norm in Equation (4), the $\ell_0$-norm $\|\cdots\|_0$ defined as non-zero components of a vector is used herein. In a dictionary-based approach, sparsifying transform is represented by a sparse decomposition of a signal into basic elements of a dictionary (called "atoms"). In the method according to the embodiment, patch-based sparse decomposition is applied. A relatively small, non-uniform sized portion of a volumetric object may be vectorized and approximated by a linear combination of atoms of a proper dictionary. Weights of this linear combination are called "sparse codes". Patch dimensionality does not matter due to patch vectorization: a two- or three-dimensional (2D or 3D) patch may be represented as a column-vector. If original dimensionality of a patch corresponds to dimensionality for the atoms, any original patch (2D or 3D) in a vectorized form may be approximated by vectorized atoms of a dictionary. Mathematically, the following optimization problem may be solved using Equation (8) below:

$$ \mathbf{z}_j = \arg\min_{\mathbf{z}_j} \left\| \boldsymbol{\pi}_j - \mathbf{D} \cdot \mathbf{z}_j \right\|_2^2 + \delta \cdot \left\| \mathbf{z}_j \right\|_0 \ \forall j $$

$$ \ldots (8) $$

where D is a dictionary transform matrix, and $\delta$ is a regularizing parameter. The column-vector $\pi_j$ in Equation (8) is a patch of a merged image of a volumetric MR object, and the corresponding j-th column-vector of sparse code $z_j$ is its sparse decomposition by the dictionary transform matrix D. The use of $\ell_0$-norm of a sparse code vector means that it is desirable to use only a minimal number of atoms in the approximation, e. g., a minimal number of non-zero weights in z. In an embodiment, a K-Singular Value Decomposition (K-SVD) method may be used to learn a dictionary on good-quality MR images of respective sizes, scales and specifics of organs in a human body to be diagnosed.

**Non-uniform sized patches**

A method according to an embodiment may be performed using various sampling techniques. A procedure of extracting patches from a merged image of an object may be performed to produce a set of volumetric portions of the merged image. An optimal aspect ratio (an anisotropy proportion with respect to the x-, y- and z- dimensions) of a patch may rely on a predominant direction of k-space sampling due to a distribution of aliasing artifacts. Since a greater patch requires more elements of a dictionary for its satisfactory approximation, the size of a patch should not be large when the size of the dictionary is restricted. If a patch covers more data along that direction in which a sampling rate is higher, a form of the patch may be a good choice. Such a strategy for covering a fixed amount of information per patch can be performed by applying non-uniform sized patches. This approach allows reconstruction of an image of a better quality than a method employing uniform sized patches.

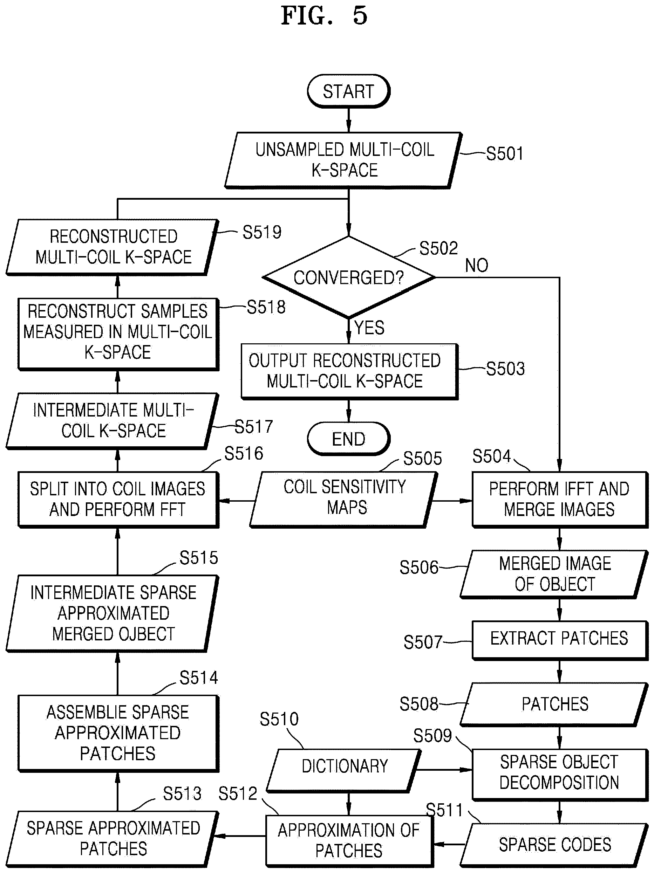

The optimization problems shown in Equations (7) and (8) may be solved using the proposed joint CS iterative algorithm that will be described with reference to FIG. 5.

FIG. 5 illustrates a dictionary-based joint CS reconstruction method according to an example embodiment.

**Dictionary-based joint CS MRI reconstruction**

Referring to FIG. 5, a highly undersampled multi-coil k-space (S501), which is used as an input of the joint CS iterative algorithm, may be initially preprocessed using the joint Split Bregman algorithm described above. Until a solution converges (S502), the following steps may be performed. First, a merged image of an object (S506) may be calculated by merging generated coil images and performing an inverse fast Fourier transform (IFFT) of the multi-coil k-space (S504). To achieve this purpose, precomputed coil sensitivity maps may be used (S505). In step S507, a set of overlapping patches (S508) may be extracted from the merged object (S506). Then, for vectorization of each 2D or 3D patch (depending on data and parameters of the algorithm), sparse object decomposition (S509) may be performed according to Equation (9), by using a proper dictionary D (S510). The sparse object decomposition can be resolved using a greedy Orthogonal Matching Pursuit algorithm. While sparse codes (S511) are obtained for each vectorized patch, approximation of patches (S512) may be performed via a linear combination of dictionary elements (S510) with sparse codes calculated as coefficients. Then, a sparse approximation of a merged object (S515) may be obtained by assembling reconstructed patches with overlapping ones (S514). In the next step S516, the merged object may be split into coil images, and the resulting coil images may be transmitted to the Fourier domain by using fast Fourier transform (FFT). These arithmetic operations (S516) may result in an intermediate multi-coil k-space (S517). Thereafter, spectral data at a sampled position in the multi-coil k-space may be restored to their measured values (S518). If the solution converges (S502), then the reconstructed multi-coil k-space (S519) may be output as the result of joint CS MRI reconstruction algorithm (S503). Otherwise, if the solution does not converge, a new iteration may begin.

**Multiband decomposition**

In a method according to an embodiment, one way to exploit a multilevel structure of data is to reconstruct a plurality of object components corresponding to different frequency bands. According to the method, a multi-coil k-space may be split into several frequency bands corresponding to low, medium, high frequencies, etc. In the splitting, several new multi-coil k-spaces appear, each of which contains its corresponding frequency band filled with measured data, whereas all other bands therein are filled with zeros. When decomposed into the bands, since a signal variation per band is significantly less than total variance of the signal, sparsity of the signal may be improved, and thus, a reconstructed signal may be of better quality. The resulting truncated k-spaces may be treated as an input for the main loop of the proposed multi-dictionary reconstruction and be processed in parallel. When all multi-coil k-spaces are reconstructed for all frequency bands, a reconstructed image may be obtained by aggregating results of the reconstruction. In a dictionary learning step, each object may be decomposed into frequency bands in the same manner as in a method of separately learning dictionaries for each frequency band. In a reconstruction step, each frequency band may be reconstructed using its corresponding dictionary.

**Multi-dictionary CS MRI reconstruction**

An essential feature of the present invention according to embodiments is multi-dictionary sparse object approximation. Dictionaries and related parameters used in the CS reconstruction (such as tolerance of the sparse approximation) may be changed during a reconstruction procedure. In the present invention, two approaches to applying multiple dictionaries in CS reconstruction are proposed.

In a first approach, dictionaries alternate with each other (See step S510 of FIG. 5) after a specific number of iterations. For example, if preliminary reconstruction is already performed, it may be very efficient to change a basis of sparse approximation, and reconstruction and emphasis of fine details of an image are required.

Another proposed approach relates to sparse object approximation using multiple dictionaries simultaneously during one iteration of CS reconstruction. In order to distinguish this approach from the above-described one, dictionary-based approximation of patches that form an image of a volumetric object will now be described in more detail.

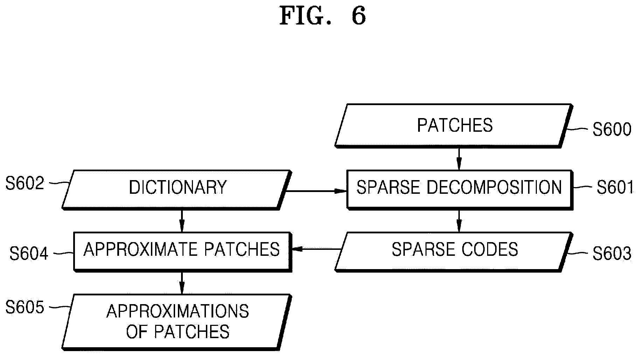

FIG. 6 illustrates single-dictionary sparse approximation of patches according to an example embodiment.

In detail, FIG. 6 illustrates a part of CS MRI reconstruction targeted on the single-dictionary approximation. Vectorized patches (S600) from volumetric portions of an object may be used as an input of dictionary-based sparse approximation. The vectorized patches may then be sparsely decomposed (S601) by a dictionary (S602) to obtain their corresponding sparse codes (S603). Then, approximation of patches (S604) may be performed up to a required accuracy (a tolerance of the sparse approximation). In FIG. 6, a result of the approximation is represented as (S650) in FIG. 6. These approximations of patches may correspond to an output of this scheme and will be further used to assemble an image of the object.

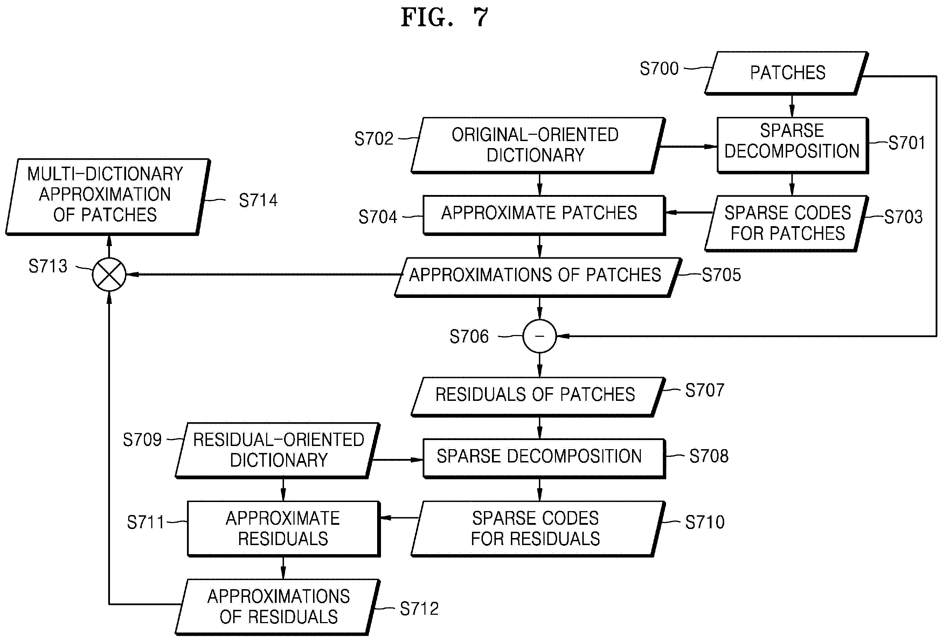

FIG. 7 illustrates multi-dictionary sparse approximation of patches according to an example embodiment.

Referring to FIG. 7, in a multi-dictionary approximation technique, a relatively simple sequence of operations are repeated to perform a multi-stage sparse approximation technique shown in FIG. 7. Vectorized patches (S700) extracted from a volumetric object may be used as an input signal. The vectorized patches may then be decomposed (S701) by a first dictionary (S702) that produces sparse codes (S703). Since an original signal is used in the first dictionary, the first dictionary is hereafter referred to as an original-oriented dictionary. Then, sparse approximations of the patches (S705) may be computed using an Orthogonal Matching Pursuit (OMP) algorithm (S704). In the next step S706, residuals of the patches (S707) may be calculated by component-wise subtracting the sparse approximations of the patches from the input patches (S700). Sparse approximation with respect to the residuals of the patches (S707) may be repeated. The residuals of the patches (S707) may be decomposed (S708) by a second dictionary (S709) that produces new sparse codes (S710). Since the second dictionary (S709) is targeted on the residuals of patches with medium- and high-frequency features, the second dictionary is hereafter called the residual-oriented dictionary. Finally, the sparse approximations of the patches (S705) (obtained by the first dictionary) and the sparse approximations of the residuals of the patches (obtained by the second dictionary) (S712) may subsequently be summed (S713) to obtain a desired multi-dictionary sparse approximation of the patches (S714). By assembling the patches, clearer MRI reconstruction may be performed compared to the above-described single-dictionary approach. Thus, it is possible to restore fine details and achieve better suppression of noise and aliasing artifacts.

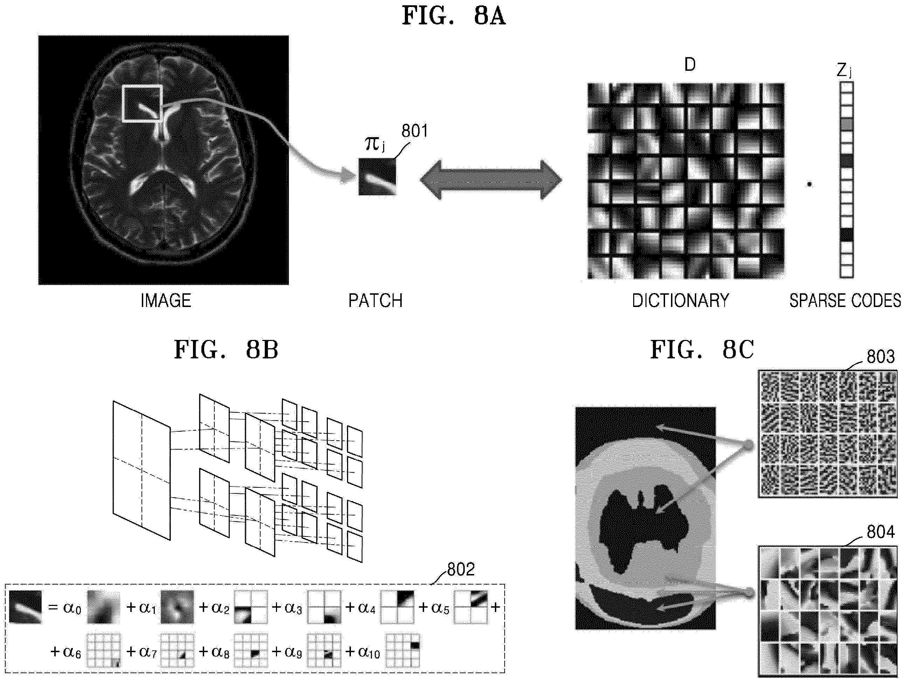

FIGS. 8A through 8C illustrate dictionary-based sparse approximations according to example embodiments.

FIG. 8A shows an example of dictionary-based sparse approximation. Furthermore, FIG 8B and 8C show different types of dictionary-based sparse approximation methods using multi-scale dictionaries and target-oriented dictionaries, respectively. In multi-scale dictionaries, a patch $\pi_j$ 801 of a size 4N × 4M can be sparse approximated by patches 802 having informative components represented within blocks of smaller sizes, e. g., 2N × 2M, N × M, etc. The rest of the blocks for the patches 802 is assumed to be zero, and in general, these informative regions may be located at various positions within the main 4N × 4M block. Since various regions of an image mainly contain features from different frequency bands, a target-oriented dictionary may include patches corresponding to different frequency bands. It can be seen that it is more efficient to apply dictionaries to various regions of a merged image depending on estimated variance of extracted patches such as low- , medium- and high-frequency patches. In FIG. 8C, different colors emphasize various regions of interest. While high-frequency patches 803 may be applied together with a dictionary D for a dark-colored region, relatively low-frequency patches 804 may be applied therewith for two regions depicted in brighter colors.

**Multi-dictionary reconstruction: dictionary learning**

Learning of dictionaries oriented towards sparse approximation of initial patches is based on a K-Singular Value Decomposition (K-SVD) algorithm [M. Aharon, M. Elad, and A. Bruckstein "K-SVD: An Algorithm for Designing Overcomplete Dictionaries for Sparse Representation," IEEE Trans. Image Process. 54, 4311-4322 (2006)]. Details of the procedure are beyond the scope of the invention according to the embodiments. An method of operation using various dictionaries is proposed herein. A learning technique is for multiple dictionaries oriented towards image patches and so-called patch- residuals, i.e. differences between original patches and their sparse approximations. While a conventional dictionary is learned on a set of original high-quality images, residual-oriented dictionaries are learned on differences between original images and their sparse approximations using an original-oriented dictionary. For example, dictionaries in steps S510 (including iterative replacement of dictionaries), S602 and S702 are original-oriented.

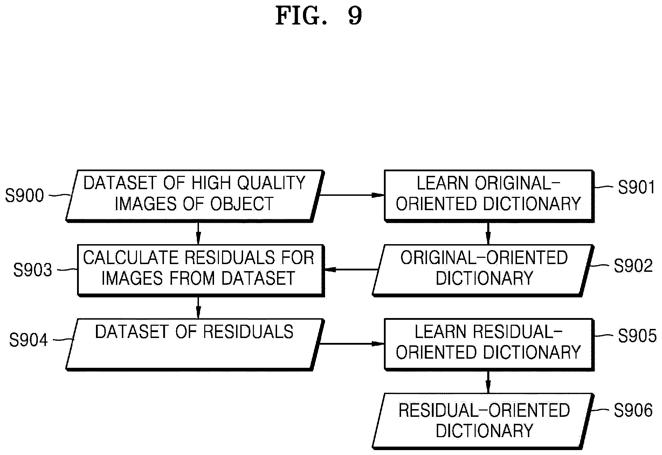

FIG. 9 illustrates a process of learning original- and residual-oriented dictionaries.

First, learning of a patch-based dictionary (S901) may be performed using a large dataset of high quality images of an object (S900). As a result, an original-oriented dictionary may be provided. According to an embodiment, a conventional K-SVD algorithm may be used for dictionary learning. In the next step S903, the computed original-oriented dictionary (S902) may be used for calculating differences between patches of original images and their sparse approximations for all images from the given dataset (S900). By calculating the differences, a dataset of residuals may be generated (S904). Then, to calculate a desired residual-oriented dictionary (S906), patch-based dictionary learning (S905) may be performed using the dataset of residuals generated in step S904.

Furthermore, both the original-oriented dictionary (S902) and the residual-oriented dictionary (S906) may be orthogonalized using a Gram-Schmidt procedure to decrease the number of atoms therein and therefore accelerate sparse-approximation.

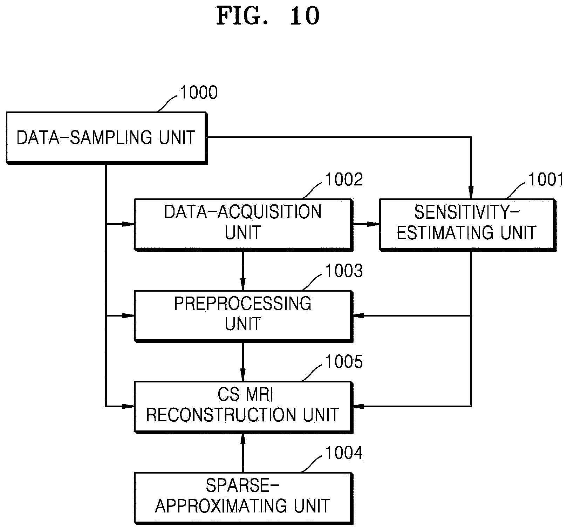

FIG. 10 illustrates a joint CS MRI reconstruction system according to an example embodiment.

**Joint CSMRI reconstruction system**

The proposed system is applicable for CS MRI reconstruction of medical images, and may include the following units (e.g., a reconstruction server) which form the system of FIG. 10:

a data-sampling unit 1000 configured to create a sampling scheme (undersampling mask) and transmit the created sampling scheme to a data-acquisition unit 1002, a preprocessing unit 1003, a sensitivity-estimating unit 1001, and a CS MRI reconstruction unit 1005;

the data-acquisition unit 1002 configured to acquire undersampled multi-coil k-spaces via multiple RF receiver coils with respect to the sampling scheme received from the data-sampling unit 1000 and then transmit the acquired multi-coil k-spaces to both the preprocessing unit 1003 and the sensitivity-estimating unit 1001;

the sensitivity-estimating unit 1001 configured to estimate coil sensitivity maps based on the sampled multi-coil k-space data, wherein spatial coordinates of sampled frequencies may be obtained from the sampling mask (prepared by the data-sampling unit 1000), and the required multi-coil k-space may be received from the data-acquisition unit 1002;

the preprocessing unit 1003 configured to fill unsampled positions in the multi-coil k-space with initial values (zeros or values provided by initialization procedures like joint Split Bregman) and transmit the initialized multi-coil k-space to the CS MRI reconstruction unit 1005, wherein k-spaces to be initialized may be received from the data-acquisition unit 1002, information on the unsampled positions may be received from the data-sampling unit 1000, and coil sensitivity maps required for the initialization algorithms may be received from the sensitivity-estimating unit 1001;

the sparse-approximating unit 1004 configured to learn the original- and/or residual-oriented dictionaries for sparse object approximation and transmits the learned dictionaries to the CS MRI reconstruction unit 1005; and

the CS MRI reconstructing unit 1005 configured to reconstruct the preprocessed multi-coil k-spaces acquired from the preprocessing unit 1003 according to a multi-dictionary joint CS MRI algorithm, wherein information about samples in the k-spaces to be reconstructed may be received from the data-sampling unit 1000, and the dictionaries and coil sensitivity maps for joint CS reconstruction may respectively be received from the sparse-approximating unit 1004 and sensitivity-estimating unit 1001.

While the data-sampling unit 1000, the sensitivity-estimating unit 1001, and the preprocessing unit 1003 constructs a block for preprocessing a multi-coil k-space, the sparse-approximating unit 1004 and the CS MRI reconstruction unit 1005 may form a block for multi-dictionary joint CS reconstruction.

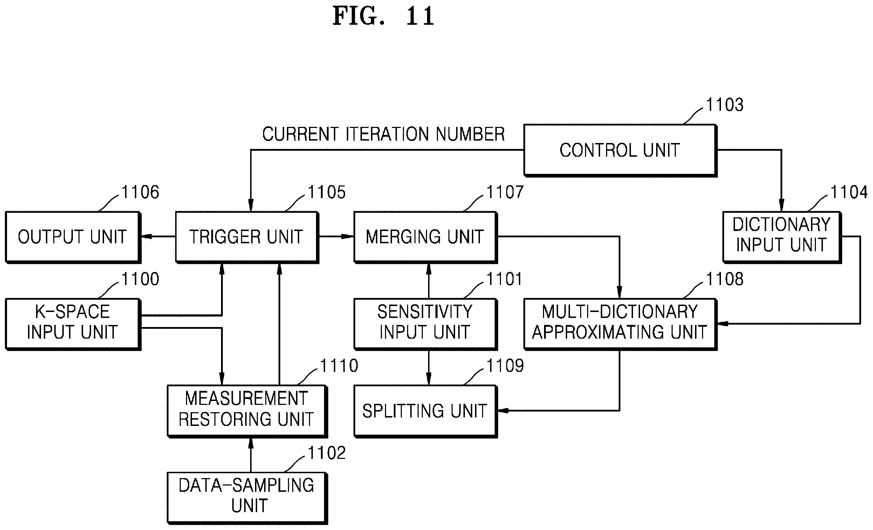

FIG. 11 illustrates a multi-dictionary CS MRI reconstruction system according to an example embodiment.

In detail, FIG. 11 illustrates a particular case of a reconstruction unit respectively in multi-dictionary approaches (dictionaries are sequentially changed during the reconstruction in this case). The multi-dictionary CS MRI reconstruction system represents an implementation of the method described above with reference to FIG. 5 and may include the following units:

a k-space input unit 1100 configured to acquire k-spaces of an object via multiple RF receiver coils, perform preprocessing on the acquired k-spaces by filling unsampled positions with several proper values (zeros or initial guesses for the joint Split Bregman), and transmit the preprocessed k-spaces to a measurement restoring unit 1110 and a trigger unit 1105.

a sensitivity input unit 1101 configured to estimate coil sensitivity maps and transmit the estimated coil sensitivity maps to a merging unit 1107 and a splitting unit 1109;

a data-sampling unit 1102 configured to create a sampling scheme (sampling mask) of k-space acquisition and transmit the created sampling scheme to the measurement restoring unit 1110;

the trigger unit 1105 configured to transmit a multi-coil k-space to be reconstructed from the measurement restoring unit 1110 to the merging unit 1107 or a k-space output unit 1106 according to a signal from the control unit 1103, which is produced via an iteration number or analysis of respective stopping criteria;

the k-space output unit 1106 configured to output and store the reconstructed multi-coil k-space;

the merging unit 1107 configured to receive the reconstructed multi-coil k-space from the trigger unit 1105, perform inverse Fourier transform on the received multi-coil k-space, and merge "coil images" by using the coil sensitivity maps acquired from the sensitivity input unit 1101, wherein the resulting merged image is transmitted to a multi-dictionary approximating unit 1108;

a dictionary input unit 1104 configured to receive a current iteration number from the control unit 1103, select proper dictionaries for dictionary-based object approximation, and transmit the selected dictionaries to the multi-dictionary approximating unit 1108;

the multi-dictionary approximating unit 1108 configured to receive the merged image from the merging unit 1107, perform sparse approximation on the merged image by using original-and/or residual-oriented dictionaries from the dictionary input unit 1104, and transmit a sparse approximation result to the sparse approximation to the splitting unit 1109;

the splitting unit 1109 configured to split the object sparse approximation acquired from the multi-dictionary approximating unit 1108 by using the coil sensitivity maps obtained from the sensitivity input unit 1101, further acquire k-spaces from images related to coils, and transmit the acquired images (a "multi-coil k-space") to the measurement restoring unit 1110;

the measurement restoring unit 1110 configured to receive the multi-coil k-space from the splitting unit 1109 and restore original values at sampled positions in k-space data, wherein the sampled positions are obtained from the data-sampling unit 1102, and transmits a result of the restoration to the trigger unit 1105; and

the control unit 1103 configured to determine two aspects of joint CS MRI reconstruction: determining a destination unit for transmitting respective k-spaces and selecting dictionaries used for a current iteration. At an initial iteration, the control unit 1103 may switch the trigger unit 1105 to receive initial data from the k-space input unit 1100 to start the data processing. In all other cases, the trigger unit 1105 may provide the k-spaces received from the measurement restoring unit 1110. Furthermore, the multi-coil k-space may be transmitted to the merging unit 1107. If a stop condition is satisfied (the number of iterations or accuracy criterion is checked for the stop condition), the control unit 1103 may switch the trigger unit 1105 to stop transmission of the multi-coil k-space from the measurement restoring unit 1110 to the merging unit 1107. In this case, the trigger unit 1105 may transmit the resulting multi-coil k-space to the k-space output unit 1106. At the same time, the control unit 1103 may notify the dictionary input unit 1104 about a current iteration number to select proper dictionaries.

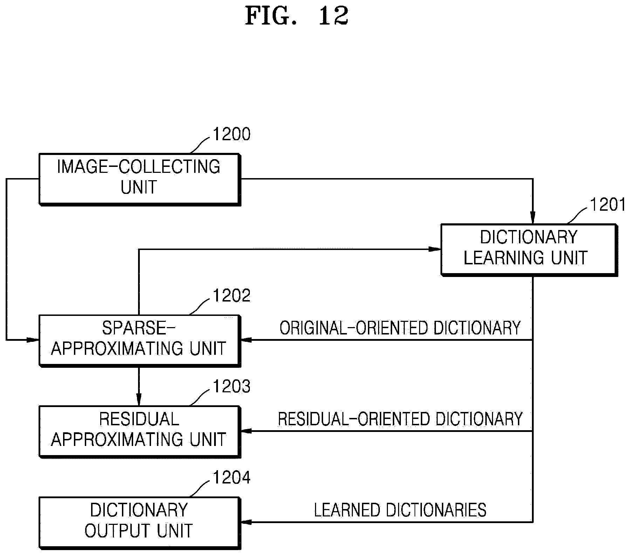

FIG. 12 illustrates a system for learning original- and residual-oriented dictionaries according to an example embodiment.

FIG. 12 illustrates an implementation of the algorithm described with reference to FIG. 9. The system may include the following units:

an image-collecting unit 1200 configured to collect a dataset of high-quality MR images of objects and transmit the collected dataset to a dictionary learning unit 1201 and a sparse-approximating unit 1202;

the dictionary learning unit 1201 configured to perform dictionary learning (e.g., by using K-SVD) and create original- and residual-oriented dictionaries according to input data, wherein all these dictionaries may be transmitted to a dictionary output unit 1204 for storing the dictionaries, and the original- and residual-oriented dictionaries may respectively be transmitted from the dictionary learning unit 1201 to the sparse-approximating unit 1202 and to a residual approximating unit 1203;

the sparse-approximating unit 1202 configured to calculate sparse approximations for all datasets of MR images from the image-collecting unit 1200 , wherein the calculation is performed with the dictionary obtained from the dictionary learning unit 1201, and residuals may be transmitted back to the dictionary learning unit 1201 and to the residual approximating unit 1203;

the residual approximating unit 1203 configured to calculate an approximation of the residuals received from the sparse-approximating unit 1202 by using the residual-oriented dictionary obtained from the dictionary leaning unit 1201; and

the dictionary output unit 1204 configured to receive and store the computed original- and residual-oriented dictionaries (the stored dictionaries are assumed to be used for the sparse approximation).

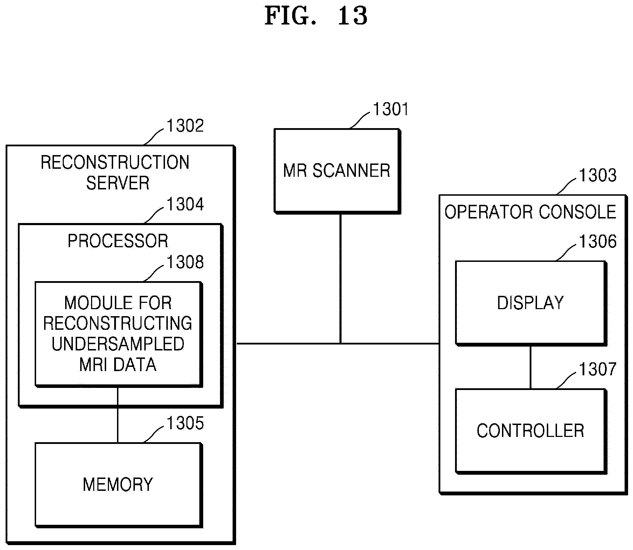

FIG. 13 illustrates an MR image processing system according to an example embodiment.

The MR image processing system of FIG. 13 may include an MR scanner 1301 configured to acquire undersampled multi-coil k-spaces based on multiple receiver coils, a reconstruction server 1302, and an operator console 1303. When undersampled multi-coil k-spaces are acquired, files may be stored in a memory 1305 of the reconstruction server 1302, together with data. An operator may run an image reconstruction program by using a controller 137 of the operator console 1303. The image reconstruction program may be executed on a processor 1304 of the reconstruction server 1302. The processor 1304 may include a module 1308 for reconstructing undersampled MRI data as its subroutine. A result of executing the program may be displayed on a display 1306 of the operator console 1303. The controller 1307 may control the result of executing the program and monitor parameters of MR images to be reconstructed and displayed.

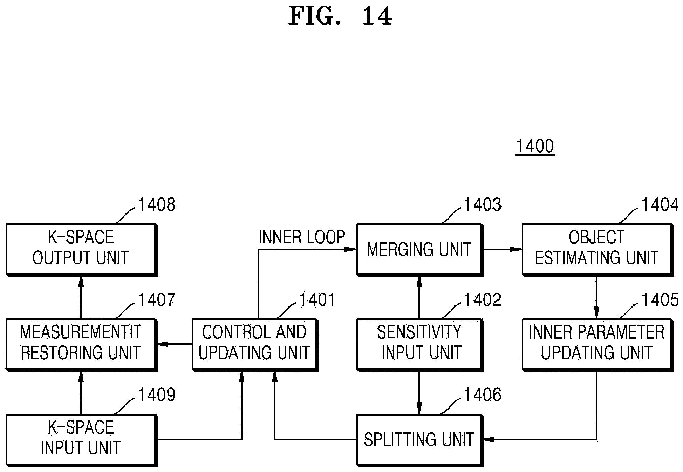

FIG. 14 illustrates a system for joint Split Bregman reconstruction according to an example embodiment of the invention.

In detail, the system 1400 of FIG. 14 may a system for initializing data by using a joint Split Bregman algorithm. FIG. 14 illustrates an implementation of the algorithm described with reference to FIG. 4. The system 1400 may include the following units.

The system 1400 may include a k-space input unit 1409 configured to acquire multi-coil k-spaces via receiver coils according to a selected sampling mask and transmit the multi-coil k-spaces and the sampling mask to a control and updating unit 1401 and a measurement restoring unit 1407.

The system 1400 may further include a sensitivity input unit 1402 configured to receive estimated sensitivity maps of receiver coils and transmit the received sensitivity maps to a merging unit 1403 and a splitting unit 1406.

The system 1400 may further include the merging unit 1403 configured to receive multi-coil k-spaces from the control and updating unit 1401, perform inverse Fourier transform on the multi-coil k-spaces, merge images obtained using the sensitivity maps (obtained from the sensitivity input unit 1402), and transmit the resulting merged image to an object estimating unit 1404.

The system 1400 may further include an object estimating unit 1404 configured to receive required parameters and the merged image from the merging unit 1403, recalculate an object sparse approximation by minimizing a respective criterion, and transmit a recalculation result to an inner parameter updating unit 1405. The object estimating unit 1404 may recalculate a sparse approximation of the object by minimizing a criterion function included an $\ell$1-norm.

The system 1400 may further include the inner parameter updating unit 1405 configured to receive sparse the updated sparse representation of the object (image) from the object estimating unit 1404, update related parameters, and transmit the updated object approximation to the splitting unit 1406.

The system 1400 may further include the splitting unit 1406 configured to split the object sparse approximation obtained from the inner parameter updating unit 1405 by using the coil sensitivity maps for this purpose (obtained from the sensitivity input unit 1402). The splitting unit 1406 may perform Fourier transform on the resulting coil images and transmit the updated multi-coil k-spaces to the control and updating unit 1401.

The control and updating unit 1401 may control processing conditions for outer and inner loops of the joint Split Bregman algorithm. If the condition for the inner loop is fulfilled, the control and updating unit 1401 may update outer parameters. If the condition for the outer loop is fulfilled, reconstructed multi-coil k-spaces may be transmitted to the measurement restoring unit 1407 while preliminary reconstructed multi-coil k-spaces may be transmitted to the merging unit 1403.

The system 1400 may further include a measurement restoring unit 1407 configured to receive the reconstructed multi-coil k-spaces from the control and updating unit 1401 and restore sampled elements of k-space data to their original (measured) values.

The system 1400 may further include a k-space output unit 1408 configured to output and store the reconstructed multi-coil k-spaces received from the measurement restoring unit 1407.

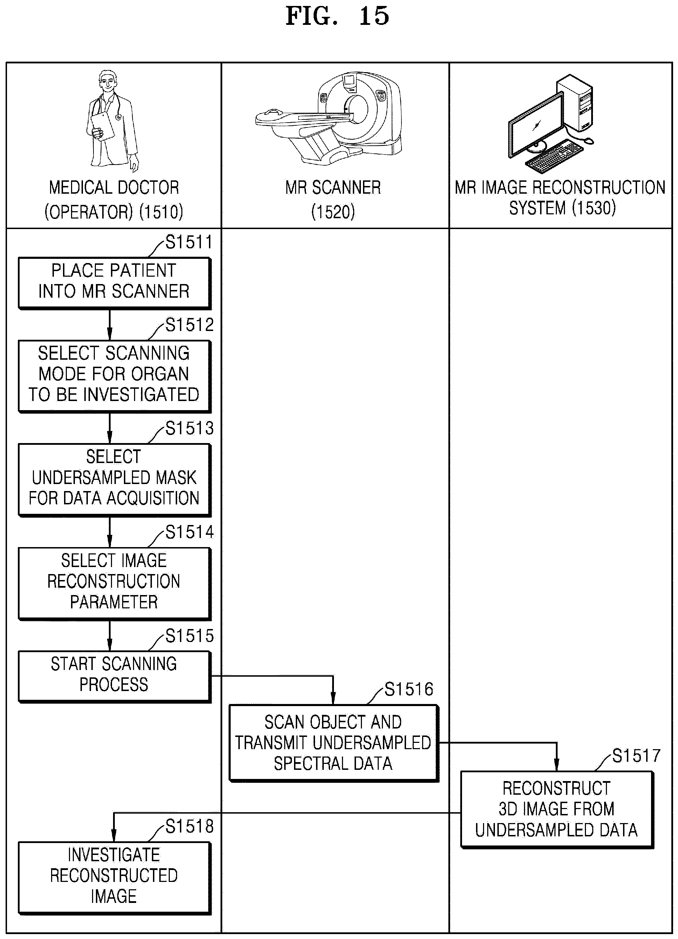

FIG. 15 illustrates a user scenario 1 according to an example embodiment.

The user scenario 1 may suppose three roles.

The three roles may include the roles of: a medical doctor or operator 1510 controlling an MR scanner1520 and an MR image reconstruction system 1530, the MR scanner 1520 for performing a measurement process and providing spectral data of measurements of an object, and the MR image reconstruction system 1530 including software and hardware components for performing an MR image reconstruction method based on undersampled spectral data.

The user scenario 1 may include the following main steps:

S1511 wherein the medical doctor 1510 places a patient into the MR scanner 1520 in such a manner as to investigate a selected patient's organ; S1512 wherein the medical doctor 1510 selects a desired scanning mode and an organ to be investigated; S1513 wherein the medical doctor 1510 selects a desired sampling mask to accelerate a scanning process; S1514 wherein the medical doctor 1510 selects a set of parameters for an image reconstruction process; S1515 wherein the medical doctor 1510 starts the scanning process; S1516 (undersampling) wherein the MR-scanner 1520 scans the patient by skipping some data to accelerate an image reconstruction process; S1517 wherein the MR image reconstruction system 1530 processes acquired data and finally reconstructs an MR image of the organ to be investigated (main steps of the algorithm implemented by the MR image reconstruction system 1530 are described in detail in the description with respect to FIG. 3); and S1518 wherein the medical doctor 1510 investigates the reconstructed MR image.

An important effect of the invention according to the embodiment is to provide a good quality MR image within a minimal time (the scanned data is undersampled, and the total scan time may be reduced by 4 to 6 times by making a predetermined compromise between "quality and spent time".

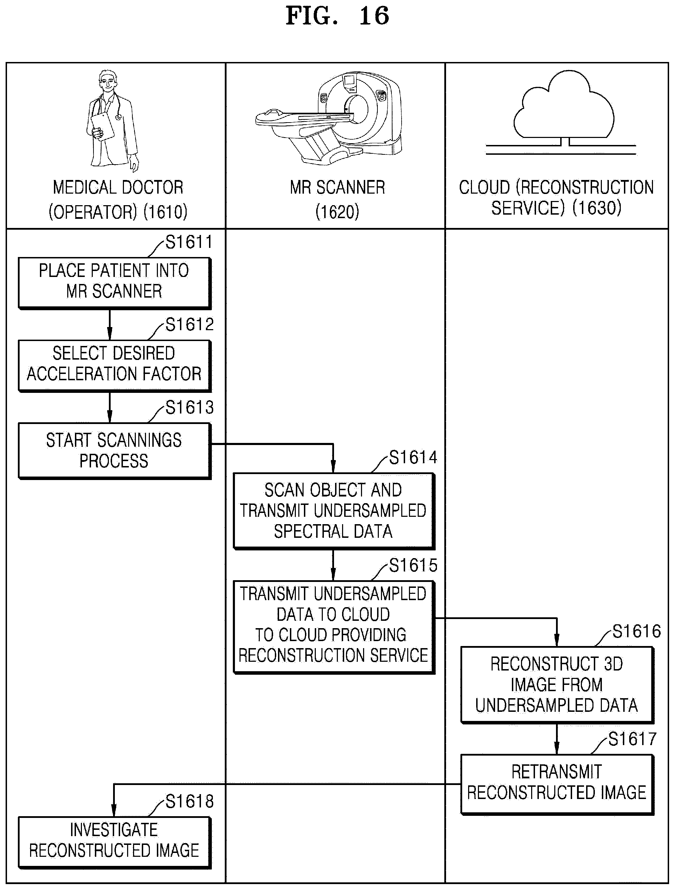

FIG. 16 illustrates a user scenario 2 according to an example embodiment.

In the user scenario 2, a reconstruction process may be performed not by hardware accessories that can be provided with an MR scanner 1620 but by a dedicated server for providing a reconstruction service. An owner of a reconstruction server or a reconstruction algorithm may be allowed access under contract with a relevant medical center. In this case, any MR scanner 1620 (that is located in the medical center) may be used without reference to the reconstruction algorithm. Reconstruction may be performed using a cloud computing technology, and in particular, using distributed computing.

The user scenario 2 may include the main steps of:S1611 wherein a medical doctor (or operator) 1610 places a patient into an MR scanner 1620 in a medical center; S1612 wherein the medical doctor (or operator) 1610 selects a desired acceleration factor; S1613 wherein the medical doctor 1610 starts a scanning process; S1614 wherein the MR scanner 1620 scans the patient and provides undersampled spectral data; S1615 wherein the MR scanner 1620 transmits the undersampled data to a reconstruction server 1630 located in a cloud and providing a reconstruction service; S1616 wherein the reconstruction server 1630 located in the cloud performs three-dimensional (3D) image reconstruction from the received undersampled data; S1617 wherein the reconstruction server 1630 located in the cloud transmits a reconstructed image back to the MR scanner 1620 in the medical center; and S1618 wherein the medical doctor 1610 in the medical center investigates the received reconstructed image.

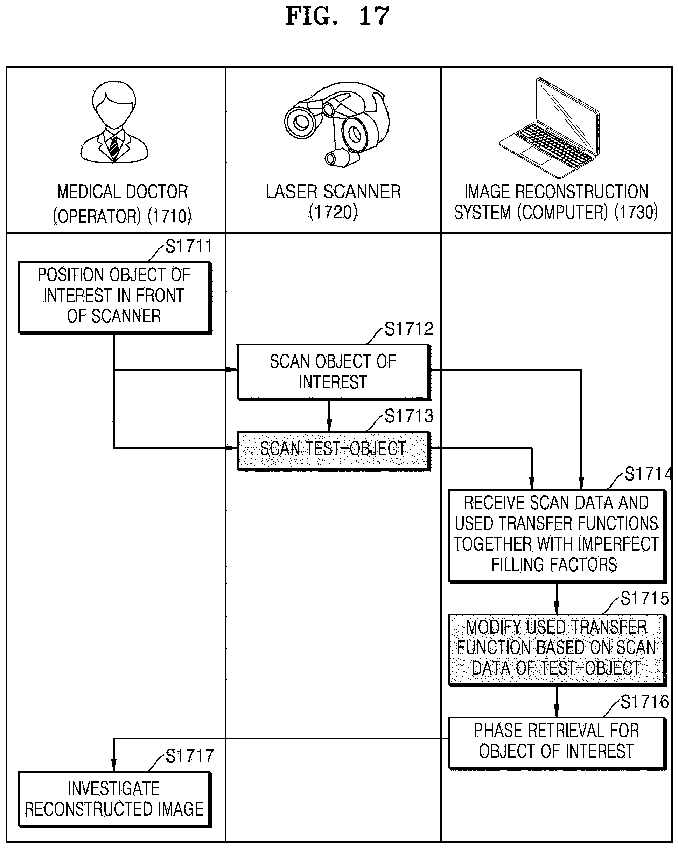

FIG. 17 illustrates a user scenario 3 according to an example outside the scope of the present invention.

The user scenario 3 may be another type of user scenario for non-destructive analysis. An object may be illuminated with a coherent laser beam, scattered radiation may be detected by a scanner, and an image of the object may be accurately reconstructed from a number of intensity measurements by a phase-retrieval algorithm on a computer. The used intensity measurement may vary due to a transfer function describing the propagation of a wave field at different distances. Accurate distances of propagation of wave field may be often encoded by specific diffractive elements, i.e., spatial light modulators (SLMs). Since filling factors of SLMs are far from perfect (depending on the cost of a SLM, the filling factor of a commercially available SLM may be from 72% to 98%), an operating Fourier spectrum may be generally undersampled. To avoid creation of ringing artifacts in reconstructed images, it may be necessary to compensate transfer functions used. For example, this may be achieved by a two-stage retrieval operation, presented in [A. Migukin et al, "Phase retrieval in 4f optical system: background compensation and sparse regularization of object with binary amplitude," Appl. Opt. 52, A269-A280 (2013)].

The user scenario 3 may assume the following three roles including: a laser scanner 1720 that illuminates an object of interest with a laser beam, detects scattered radiation from the object, and receives a number of intensity measurements corresponding to different transfer functions (encoded by an SLM), an image reconstruction system 1730 in which software necessary for image reconstruction is installed, and a user (operator) 1710 who controls the image reconstruction system 1730 and investigates a reconstructed image.

The user scenario 3 may include the main steps of: S1711 wherein the user (operator) 1710 positions an object of interest (or a fraction thereof) in front of the laser scanner 1720; S1712 wherein the user (operator) 1710 uses the laser scanner 1720 to scan the object of interest (or fraction thereof); S1713 wherein the user (operator) 1710 positions the laser scanner 1720 in front of a test-object and scans the test-object by using the laser scanner 1720; S1714 wherein an image reconstruction system (a computer) 1730 receives scan data of the known test object and the object of interest; S1715 wherein the image reconstruction system (computer) modifies a transfer function based on the scan data of the test object; S1716 wherein the image reconstruction system reconstructs an image of the object of interest from the scan data of the object of interest by using the modified transfer function; and S1717 wherein the user (operator) investigates the reconstructed image of the object.

A technical effect of the invention according to the embodiment is to provide a good quality MR image within a minimal time (since the acquired data is undersampled, a total acquisition time is reduced by about 4 to about 6 times proportionally). In other words, according to the present invention, it is possible to obtain an MR image of similar or better quality while significantly accelerating data acquisition, compared to when using known solutions. The present specification describes principles of the present disclosure and sets forth embodiments thereof to clarify the scope of the present disclosure and to allow those of ordinary skill in the art to implement the embodiments. The present embodiments may have different forms and should not be construed as being limited to the descriptions set forth herein.

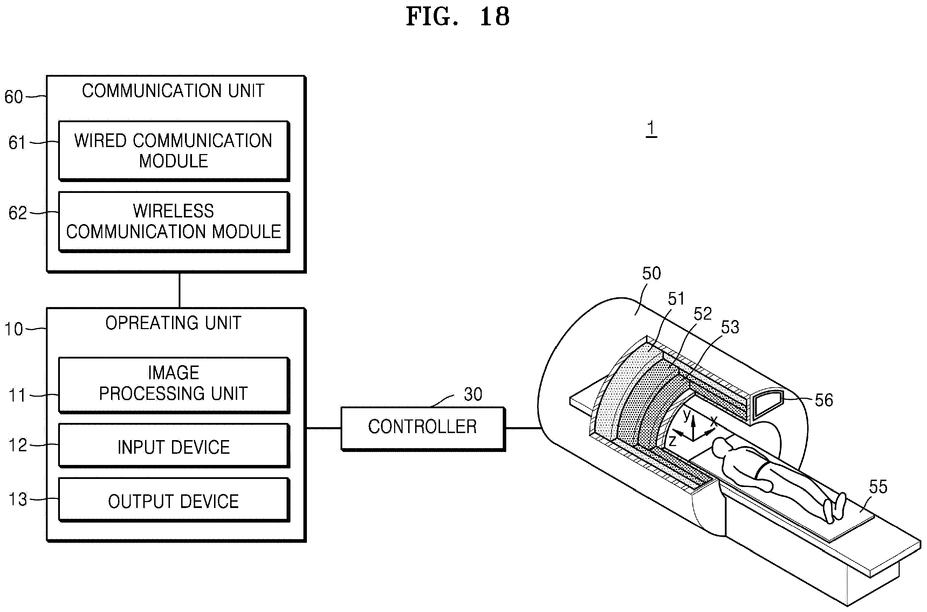

FIG. 18 is a schematic diagram of an MRI system 1.

Referring to FIG. 18, the MRI system 1 may include an operating unit 10, a controller 30, and a scanner 50. The controller 30 may be independently separated from the operating unit 10 and the scanner 50. Furthermore, the controller 30 may be separated into a plurality of sub-components and incorporated into the operating unit 10 and the scanner 50 in the MRI system 1. Operations of the components in the MRI system 1 will now be described in detail.

The scanner 50 may be formed to have a cylindrical shape (e.g., a shape of a bore) having an empty inner space into which an object may be inserted. A static magnetic field and a gradient magnetic field are created in the inner space of the scanner 50, and an RF signal is emitted toward the inner space.

The scanner 50 may include a static magnetic field generator 51, a gradient magnetic field generator 52, an RF coil unit 53, a table 55, and a display 56. The static magnetic field generator 51 creates a static magnetic field for aligning magnetic dipole moments of atomic nuclei of the object in a direction of the static magnetic field. The static magnetic field generator 51 may be formed as a permanent magnet or superconducting magnet using a cooling coil.

The gradient magnetic field generator 52 is connected to the controller 30 and generates a gradient magnetic field by applying a gradient to a static magnetic field in response to a control signal received from the controller 30. The gradient magnetic field generator 52 includes X, Y, and Z coils for generating gradient magnetic fields in X-, Y-, and Z-axis directions crossing each other at right angles and generates a gradient signal according to a position of a region being imaged so as to differently induce resonance frequencies according to regions of the object.

The RF coil unit 53 connected to the controller 30 may emit an RF signal toward the object in response to a control signal received from the controller 30 and receive an MR signal emitted from the object. In detail, the RF coil unit 53 may transmit, toward atomic nuclei of the object having precessional motion, an RF signal having the same frequency as that of the precessional motion, stop transmitting the RF signal, and then receive an MR signal emitted from the object.

The RF coil unit 53 may be formed as a transmitting RF coil for generating an electromagnetic wave having an RF corresponding to the type of an atomic nucleus, a receiving RF coil for receiving an electromagnetic wave emitted from an atomic nucleus, or one transmitting/receiving RF coil serving both functions of the transmitting RF coil and receiving RF coil. Furthermore, in addition to the RF coil unit 53, a separate coil may be attached to the object. Examples of the separate coil may include a head coil, a spine coil, a torso coil, and a knee coil according to a region being imaged or to which the separate coil is attached.

The display 56 may be disposed outside and/or inside the scanner 50. The display 56 is also controlled by the controller 30 to provide a user or the object with information related to medical imaging.

Furthermore, the scanner 50 may include an object monitoring information acquisition unit (not shown) configured to acquire and transmit monitoring information about a state of the object. For example, the object monitoring information acquisition unit may acquire monitoring information related to the object from a camera (not shown) for capturing images of a movement or position of the object, a respiration measurer (not shown) for measuring the respiration of the object, an ECG measurer for measuring the electrical activity of the heart, or a temperature measurer for measuring a temperature of the object and transmit the acquired monitoring information to the controller 30. The controller 30 may in turn control an operation of the scanner 50 based on the monitoring information. Operations of the controller 30 will now be described in more detail.

The controller 30 may control overall operations of the scanner 50.

The controller 30 may control a sequence of signals formed in the scanner 50. The controller 30 may control the gradient magnetic field generator 52 and the RF coil unit 53 according to a pulse sequence received from the operating unit 10 or a designed pulse sequence.

A pulse sequence may include all pieces of information required to control the gradient magnetic field generator 52 and the RF coil unit 53. For example, the pulse sequence may include information about a strength, a duration, and application timing of a pulse signal applied to the gradient magnetic field generator 52.

The controller 30 may control a waveform generator (not shown) for generating a gradient wave, i.e., an electrical pulse according to a pulse sequence and a gradient amplifier (not shown) for amplifying the generated electrical pulse and transmitting the same to the gradient magnetic field generator 52. Thus, the controller 30 may control formation of a gradient magnetic field by the gradient magnetic field generator 52.

Furthermore, the controller 30 may control an operation of the RF coil unit 53. For example, the controller 30 may supply an RF pulse having a resonance frequency to the RF coil unit 30 that emits an RF signal toward the object, and receive an MR signal received by the RF control unit 53. In this case, the controller 30 may adjust emission of an RF signal and reception of an MR signal according to an operating mode by controlling an operation of a switch (e.g., a T/R switch) for adjusting transmitting and receiving directions of the RF signal and the MR signal based on a control signal.

The controller 30 may control a movement of the table 55 where the object is placed. Before MRI is performed, the controller 30 may move the table 55 according to which region of the object is to be imaged.

The controller 30 may also control the display 56. For example, the controller 30 control the on/off state of the display 56 or a screen to be output on the display 56 according to a control signal.

The controller 30 may be formed as an algorithm for controlling operations of the components in the MRI system 1, a memory (not shown) for storing data in the form of a program, and a processor for performing the above-described operations by using the data stored in the memory. In this case, the memory and the processor may be implemented as separate chips. Alternatively, the memory and processor may be incorporated into a single chip.

The operating unit 10 may control overall operations of the MRI system 1 and include an image processing unit 11, an input device 12, and an output device 13.

The image processing unit 11 may control the memory to store an MR signal received from the controller 30, and generate image data with respect to the object from the stored MR signal by applying an image reconstruction technique by using an image processor.

For example, if a k space (for example, also referred to as a Fourier space or a frequency space) of the memory is filled with digital data to complete k-space data, the image processing unit 11 may reconstruct image data from the k-space data by applying various image reconstruction techniques (e.g., by performing inverse Fourier transform on the k-space data) by using the image processor.

Furthermore, the image processing unit 11 may perform various signal processing operations on MR signals in parallel. For example, image processing unit 11 may perform signal processing on a plurality of MR signals received via a multi-channel RF coil in parallel so as to convert the plurality MR signals into image data. In addition, the image processing unit 11 may store not only the image data in the memory, or the controller 30 may store the same in an external server via a communication unit 60 as will be described below.

The input device 12 may receive, from the user, a control command for controlling the overall operations of the MRI system 1. For example, the input device 12 may receive, from the user, object information, parameter information, a scan condition, and information about a pulse sequence. The input device 12 may be a keyboard, a mouse, a track ball, a voice recognizer, a gesture recognizer, a touch screen, or any other input device.

The output device 13 may output image data generated by the image processing unit 11. The output device 13 may also output a user interface (UI) configured so that the user may input a control command related to the MRI system 1. The output device 13 may be formed as a speaker, a printer, a display, or any other output device.

Furthermore, although FIG. 18 shows that the operating unit 10 and the controller 30 are separate components, the operating unit 10 and the controller 30 may be included in a single device as described above. Furthermore, processes respectively performed by the operating unit 10 and the controller 30 may be performed by another component. For example, the image processing unit 11 may convert an MR signal received from the controller 30 into a digital signal, or the controller 30 may directly perform the conversion of the MR signal into the digital signal.

The MRI system 1 may further include a communication unit 60 and be connected to an external device (not shown) such as a server, a medical apparatus, and a portable device (e.g., a smartphone, a tablet PC, a wearable device, etc.) via the communication unit 60.

The communication unit 60 may include at least one component that enables communication with an external device. For example, the communication unit 60 may include at least one of a local area communication module (not shown), a wired communication module 61, and a wireless communication module 62.

The communication unit 60 may receive a control signal and data from an external device and transmit the received control signal to the controller 30 so that the controller 30 may control the MRI system 1 according to the received signal.

Alternatively, by transmitting a control signal to an external device via the communication unit 60, the controller 30 may control the external device according to the control signal.

For example, the external device may process data of the external device according to a control signal received from the controller 30 via the communication unit 60.

A program for controlling the MRI system 1 may be installed on the external device and may include instructions for performing some or all of the operations of the controller 30.

The program may be preinstalled on the external device, or a user of the external device may download the program from a server providing an application for installation. The server providing an application may include a recording medium having the program recorded thereon.

Embodiments may be implemented through non-transitory computer-readable recording media having recorded thereon computer-executable instructions and data. The instructions may be stored in the form of program codes, and when executed by a processor, generate a predetermined program module to perform a specific operation. Furthermore, when being executed by the processor, the instructions may perform specific operations according to the embodiments.

While one or more embodiments have been described with reference to the figures, it will be understood by those of ordinary skill in the art that various changes in form and details may be made therein. The scope of the present disclosure is defined by the following claims. Accordingly, the above embodiments and all aspects thereof are examples only and are not limiting.

**Claims**

1. A magnetic resonance, MR, image processing apparatus (100, 1302, 1530, 1630, 1730) comprising a processor (110, 1304) and a memory (120, 1305) connected to the processor (110, 1304), wherein the processor (110, 1304) is configured to

   obtain partially sampled multi-coil k-space data with respect to an object; and
   obtain a reconstructed image of the object by reconstructing the partially sampled multi-coil k-space data based on using a first dictionary to identify approximations of patches (802, 803, 804) for the object, and a second dictionary, obtained based on a residual between a pre-obtained MR image of the object and an approximation of the pre-obtained MR image which is obtained based on the first dictionary, to identify approximations of residuals between the patches (802, 803, 804) and the approximations of the patches (802, 803, 804), wherein the residual between the pre-obtained MR image of the object and the approximation of the pre-obtained MR image is obtained by calculating differences between the pre-obtained MR image and the approximation of the pre-obtained MR image.

2. The MR image processing apparatus (100, 1302, 1530, 1630, 1730) of claim 1, wherein the approximation of the pre-obtained MR image may be a sparse approximation.

3. The MR image processing apparatus (100, 1302, 1530, 1630, 1730) of claim 1, wherein the processor (110, 1304) is further configured to obtain a first image based on the partially sampled multi-coil k-space data and obtain the reconstructed image by reconstructing the multi-coil k-space data based on the first dictionary, the second dictionary, and the patches (802, 803, 804) which are non-uniform sized and obtained from the first image.

4. The MR image processing apparatus (100, 1302, 1530, 1630, 1730) of claim 1, wherein the processor (110, 1304) is further configured to obtain the reconstructed image by reconstructing the multi-coil k-space data based on the patches (802, 803, 804) corresponding to different frequency bands, which are obtained based on the partially sampled multi-coil k-space data, the first dictionary, and the second dictionary.

5. The MR image processing apparatus (100, 1302, 1530, 1630, 1730) of claim 1, further comprising an operator console,
   wherein the operator console comprises a display configured to visualize the reconstructed image.

6. The MR image processing apparatus (100, 1302, 1530, 1630, 1730) of claim 5, wherein the operator console further comprises a controller (30, 1307), and
   wherein the controller (30, 1307) is configured to control the MR image processing apparatus (100, 1302, 1530, 1630, 1730).

7. The MR image processing apparatus (100, 1302, 1530, 1630, 1730) of claim 1, further comprising an MR scanner (50, 1520, 1620, 1720) configured to obtain the partially sampled multi-coil k-space data.

8. The MR image processing apparatus (100, 1302, 1530, 1630, 1730) of claim 1, wherein the processor (110, 1304) is further configured to decompose the obtained partially sampled multi-coil k-space data into a plurality of frequency bands and obtain the reconstructed image by reconstructing a plurality of further partially sampled multi-coil k-space data corresponding to the plurality of frequency bands based on the first and second dictionaries.

9.  The MR image processing apparatus (100, 1302, 1530, 1630, 1730) of claim 1, wherein the processor (110, 1304) is further configured to preprocess the partially sampled multi-coil k-space data and reconstruct the preprocessed multi-coil k-space data based on the first and second dictionaries.

10. The MR image processing apparatus (100, 1302, 1530, 1630, 1730) of claim 9, wherein the processor (110, 1304) is further configured to preprocess the partially sampled multi-coil k-space data by filling unsampled positions in the partially sampled multi-coil k-space data with initial guesses and performing coil compression on the partially sampled multi-coil k-space data.

11. A method of reconstructing a magnetic resonance, MR, image, the method comprising steps of:

    obtaining partially sampled multi-coil k-space data with respect to an object; and
    obtaining a reconstructed image of the object by reconstructing the multi-coil k-space data based on a first dictionary to identify approximations of patches (802, 803, 804) for the object, and a second dictionary, obtained based on a residual between a pre-obtained MR image of the object and an approximation of the pre-obtained MR image which is approximated based on the first dictionary, to identify approximations of residuals between the patches (802, 803, 804) and the approximations of the patches (802, 803, 804), wherein the residual between the pre-obtained MR image of the object and the approximation of the image is calculated as differences between the pre-obtained MR image and the approximation of the pre-obtained MR image.

12. The method of claim 11, wherein the step of obtaining the reconstructed image of the object comprises steps of:

    obtaining a first image based on the partially sampled multi-coil k-space data; and
    obtaining the reconstructed image by reconstructing the multi-coil k-space data based on the first dictionary, the second dictionary, and the patches (802, 803, 804 ) which are non-uniform sized and obtained from the first image.

13. A computer-readable recording medium having stored therein a program which, when performed on a processor (110, 1304), performs the steps of the method of any one of claims 11 and 12.

**Patentansprüche**

1.  Magnetresonanzbildverarbeitungsvorrichtung, MR-Bildverarbeitungsvorrichtung, (100, 1302, 1530, 1630, 1730), umfassend einen Prozessor (110, 1304) und einen mit dem Prozessor (110, 1304) verbundenen Speicher (120, 1305), wobei der Prozessor (110, 1304) dazu ausgestaltet ist,
    teilweise abgetastete Mehrspulen-k-Raum-Daten in Bezug auf ein Objekt zu erhalten; und ein rekonstruiertes Bild des Objekts durch Rekonstruieren der teilweise abgetasteten Mehrspulen-k-Raum-Daten basierend auf der Verwendung eines ersten Wörterbuchs, um Approximationen von Patches (802, 803, 804) für das Objekt zu identifizieren, und eines zweiten Wörterbuchs zu erhalten, das basierend auf einem Residuum zwischen einem zuvor erhaltenen MR-Bild des Objekts und einer Approximation des zuvor erhaltenen MR-Bilds erhalten wird, die basierend auf dem ersten Wörterbuch erhalten wird, um Approximationen von Residuen zwischen den Patches (802, 803, 804) und den Approximationen der Patches (802, 803, 804) zu identifizieren, wobei das Residuum zwischen dem zuvor erhaltenen MR-Bild des Objekts und der Approximation des zuvor erhaltenen MR-Bilds durch Berechnen der Unterschiede zwischen dem zuvor erhaltenen MR-Bild und der Approximation des zuvor erhaltenen MR-Bilds berechnet wird.

2.  MR-Bildverarbeitungsvorrichtung (100, 1302, 1530, 1630, 1730) nach Anspruch 1, wobei die Approximation des zuvor erhaltenen MR-Bilds eine spärliche Approximation sein kann.

3.  MR-Bildverarbeitungsvorrichtung (100, 1302, 1530, 1630, 1730) nach Anspruch 1, wobei the Prozessor (110, 1304) ferner dazu ausgestaltet ist, ein erstes Bild basierend auf den teilweise abgetasteten Mehrspulen-k-Raum-Daten zu erhalten
    und das rekonstruierte Bild durch Rekonstruieren der Mehrspulen-k-Raum-Daten basierend auf dem ersten Wörterbuch, dem zweiten Wörterbuch und den Patches (802, 803, 804) zu erhalten, die keine einheitliche Größe haben und aus dem ersten Bild erhalten werden.

4.  MR-Bildverarbeitungsvorrichtung (100, 1302, 1530, 1630, 1730) nach Anspruch 1, wobei der Prozessor (110, 1304) ferner dazu ausgestaltet ist, das rekonstruierte Bild durch Rekonstruieren der Mehrspulen-k-Raum-Daten basierend auf den Patches (802, 803, 804) entsprechend unterschiedlichen Frequenzbändern zu erhalten, die basierend auf den teilweise abgetasteten Mehrspulen-k-Raum-Daten, dem ersten Wörterbuch und dem zweiten Wörterbuch erhalten werden.

5. MR-Bildverarbeitungsvorrichtung (100, 1302, 1530, 1630, 1730) nach Anspruch 1, ferner umfassend ein Bedienpult, wobei das Bedienpult eine Anzeige umfasst, die dazu ausgestaltet ist, das rekonstruierte Bild zu visualisieren.

6. MR-Bildverarbeitungsvorrichtung (100, 1302, 1530, 1630, 1730) nach Anspruch 5, wobei das Bedienpult ferner eine Steuerung (30, 1307) umfasst, und
wobei die Steuerung (30, 1307) dazu ausgestaltet ist, die MR-Bildverarbeitungsvorrichtung (100, 1302, 1530, 1630, 1730) zu steuern.

7. MR-Bildverarbeitungsvorrichtung (100, 1302, 1530, 1630, 1730) nach Anspruch 1, ferner umfassend einen MR-Scanner (50, 1520, 1620, 1720), der dazu ausgestaltet ist, die teilweise abgetasteten Mehrspulen-k-Raum-Daten zu erhalten.

8. MR-Bildverarbeitungsvorrichtung (100, 1302, 1530, 1630, 1730) nach Anspruch 1, wobei der Prozessor (110, 1304) ferner dazu ausgestaltet ist, die erhaltenen teilweise abgetasteten Mehrspulen-k-Raum-Daten in eine Mehrzahl von Frequenzbändern zu zerlegen und
das rekonstruierte Bild durch Rekonstruieren einer Mehrzahl weiterer teilweise abgetasteter Mehrspulen-k-Raum-Daten entsprechend der Mehrzahl von Frequenzbändern basierend auf dem ersten und dem zweiten Wörterbuch zu erhalten.

9. MR-Bildverarbeitungsvorrichtung (100, 1302, 1530, 1630, 1730) nach Anspruch 1, wobei der Prozessor (110, 1304) ferner dazu ausgestaltet ist, die teilweise abgetasteten Mehrspulen-k-Raum-Daten vorzuverarbeiten und die vorverarbeiteten Mehrspulen-k-Raum-Daten basierend auf dem ersten und dem zweiten Wörterbuch zu rekonstruieren.

10. MR-Bildverarbeitungsvorrichtung (100, 1302, 1530, 1630, 1730) nach Anspruch 9, wobei der Prozessor (110, 1304) ferner dazu ausgestaltet ist, die teilweise abgetasteten Mehrspulen-k-Raum-Daten durch Füllen unabgetasteter Positionen in den teilweise abgetasteten Mehrspulen-k-Raum-Daten mit Anfangsschätzungen vorzuverarbeiten und eine Spulenkompression an den teilweise abgetasteten Mehrspulen-k-Raum-Daten durchzuführen.

11. Verfahren zum Rekonstruieren eines Magnetresonanzbilds, MR-Bilds, wobei das Verfahren die folgenden Schritte umfasst:

Erhalten teilweise abgetasteter Mehrspulen-k-Raum-Daten in Bezug auf ein Objekt; und
Erhalten eines rekonstruierten Bilds des Objekts durch Rekonstruieren der Mehrspulen-k-Raum-Daten basierend auf einem ersten Wörterbuch, um Approximationen von Patches (802, 803, 804) für das Objekt zu identifizieren, und einem zweiten Wörterbuch, das basierend auf einem Residuum zwischen einem zuvor erhaltenen MR-Bild des Objekts und einer Approximation des zuvor erhaltenen MR-Bilds erhalten wird, die basierend auf dem ersten Wörterbuch approximiert wird, um Approximationen von Residuen zwischen den Patches (802, 803, 804) und den Approximationen der Patches (802, 803, 804) zu identifizieren, wobei das Residuum zwischen dem zuvor erhaltenen MR-Bild des Objekts und der Approximation des Bilds als Unterschiede zwischen dem zuvor erhaltenen MR-Bild und der Approximation des zuvor erhaltenen MR-Bilds berechnet wird.

12. Verfahren nach Anspruch 11, wobei der Schritt des Erhaltens des rekonstruierten Bilds des Objekts die folgenden Schritte umfasst:

Erhalten eines ersten Bilds basierend auf den teilweise abgetasteten Mehrspulen-k-Raum-Daten; und
Erhalten des rekonstruierten Bilds durch Rekonstruieren der Mehrspulen-k-Raum-Daten basierend auf dem ersten Wörterbuch, dem zweiten Wörterbuch und den Patches (802, 803, 804), die keine einheitliche Größe haben und aus dem ersten Bild erhalten werden.

13. Computerlesbares Aufzeichnungsmedium mit einem darin gespeicherten Programm, das, wenn es auf einem Prozessor (110, 1304) ausgeführt wird, die Schritte des Verfahrens nach einem der Ansprüche 11 und 12 ausführt.

**Revendications**

1. Appareil de traitement d'image de résonance magnétique, MR, (100, 1302, 1530, 1630, 1730) comprenant un processeur (110, 1304) et une mémoire (120, 1305) connectée au processeur (110, 1304), le processeur (110, 1304) étant configuré pour obtenir des données de k-espace multibobine partiellement échantillonné relativement à un objet ; et
obtenir une image reconstruite de l'objet en reconstruisant les données de k-espace multibobine partiellement échantillonné basé sur l'utilisation d'un premier dictionnaire pour identifier des approximations de patches (802, 803, 804) pour l'objet, et d'un deuxième dictionnaire, obtenu basé sur un résidu entre une image MR pré-obtenue de l'objet et une approximation de l'image MR pré-obtenue qui est obtenue sur la base du premier dictionnaire, pour identifier des approximations de résidus entre les patches (802, 803, 804) et les approximations des patches (802, 803, 804), le résidu entre l'image MR pré-obtenue de l'objet et l'approximation de l'image MR pré-obtenue étant obtenu par le calcul des différences entre l'image MR pré-obtenue et l'approximation de l'image MR pré-obtenue.

2. Appareil de traitement d'image MR (100, 1302, 1530, 1630, 1730) selon la revendication 1, l'approximation de l'image MR pré-obtenue pouvant être une approximation parcimonieuse.

3. Appareil de traitement d'image MR (100, 1302, 1530, 1630, 1730) selon la revendication 1, le processeur (110, 1304) étant configuré en outre pour obtenir une première image sur la base des données de k-espace multibobine partiellement échantillonné et pour obtenir l'image reconstruite par reconstruction des données de k-espace multibobine sur la base du premier dictionnaire, du deuxième dictionnaire et des patches (802, 803, 804) qui sont dimensionnés non uniformément et obtenus de la première image.

4. Appareil de traitement d'image MR (100, 1302, 1530, 1630, 1730) selon la revendication 1, le processeur (110, 1304) étant configuré en outre pour obtenir l'image reconstruite par reconstruction des données de k-espace multibobine sur la base des patches (802, 803, 804) correspondant à différentes bandes de fréquence, lesquelles sont obtenues sur la base des données de k-espace multibobine partiellement échantillonné, du premier dictionnaire et du deuxième dictionnaire.

5. Appareil de traitement d'image MR (100, 1302, 1530, 1630, 1730) selon la revendication 1, comprenant en outre une console opérateur,
la console opérateur comprenant un écran configuré pour afficher l'image reconstruite.

6. Appareil de traitement d'image MR (100, 1302, 1530, 1630, 1730) selon la revendication 5, la console opérateur comprenant en outre un contrôleur (30, 1307), et
le contrôleur (30, 1307) étant configuré pour commander l'appareil de traitement d'image MR (100, 1302, 1530, 1630, 1730).

7. Appareil de traitement d'image MR (100, 1302, 1530, 1630, 1730) selon la revendication 1, comprenant en outre un scanner MR (50, 1520, 1620, 1720), configuré pour obtenir les données de k-espace multibobine partiellement échantillonné.

8. Appareil de traitement d'image MR (100, 1302, 1530, 1630, 1730) selon la revendication 1, le processeur (110, 1304) étant configuré en outre pour décomposer les données de k-espace multibobine partiellement échantillonné en une pluralité de bandes de fréquence et pour
obtenir l'image reconstruite par reconstruction d'une pluralité de données de k-espace multibobine partiellement échantillonné correspondant à la pluralité de bandes de fréquence sur la base des premier et deuxième dictionnaires.

9. Appareil de traitement d'image MR (100, 1302, 1530, 1630, 1730) selon la revendication 1, le processeur (110, 1304) étant configuré en outre pour prétraiter les données de k-espace multibobine partiellement échantillonné et pour reconstruire les données de k-espace multibobine prétraitées sur la base des premier et deuxième dictionnaires.

10. Appareil de traitement d'image MR (100, 1302, 1530, 1630, 1730) selon la revendication 9, le processeur (110, 1304) étant configuré en outre pour prétraiter les données de k-espace multibobine partiellement échantillonné en remplissant des positions non échantillonnées dans les données de k-espace multibobine partiellement échantillonné avec hypothèses initiales et en exécutant une compression de bobines sur les données de k-espace multibobine partiellement échantillonné.

EP 3 346 443 B1

11. Procédé de reconstruction d'une image de résonance magnétique, MR, le procédé comprenant les étapes de :

obtenir des données de k-espace multibobine partiellement échantillonné relativement à un objet ; et
obtenir une image reconstruite de l'objet en reconstruisant les données de k-espace multibobine partiellement échantillonné sur la base d'un premier dictionnaire pour identifier des approximations de patches (802, 803, 804) pour l'objet, et d'un deuxième dictionnaire, obtenu sur la base d'un résidu entre une image MR pré-obtenue de l'objet et une approximation de l'image MR pré-obtenue qui est approximée sur la base du premier dictionnaire, pour identifier des approximations de résidus entre les patches (802, 803, 804) et les approximations des patches (802, 803, 804),
le résidu entre l'image MR pré-obtenue de l'objet et l'approximation de l'image étant calculé comme différences entre l'image MR pré-obtenue et l'approximation de l'image MR pré-obtenue.

12. Procédé selon la revendication 11, les étapes d'obtention de l'image reconstruite de l'objet comprenant les étapes de :

obtenir une première image basé sur les données de k-espace multibobine partiellement échantillonné ; et
obtenir l'image reconstruite par reconstruction des données de k-espace multibobine sur la base du premier dictionnaire, du deuxième dictionnaire et des patches (802, 803, 804) qui sont dimensionnés non uniformément et obtenus de la première image.

13. Support d'enregistrement lisible par ordinateur, sur lequel est enregistré un programme, lequel, lorsqu'il est exécuté par un processeur (110, 1304), exécute les étapes du procédé selon l'une quelconque des revendications 11 et 12.

# FIG. 1

<u>100</u>

RECONSTRUCTION SERVER

PROCESSOR ⟋110

MEMORY ⟋120

# FIG. 2

START

ACQUIRE UNDERSAMPLED MULTI-COIL K-SPACE — S110

OBTAIN RECONSTRUCTED IMAGE OF OBEJECT
BY USING FIRST AND SECOND DICTIONARIES — S120

END

# FIG. 3A

```
                    ┌─────────┐
                    │  START  │
                    └─────────┘
                         │
                         ▼
┌──────────────────────────────────────────────────────┐
│ ACQUIRE UNDERSAMPLED MULTI-COIL K-SPACE DATA           │──── S210
└──────────────────────────────────────────────────────┘
                         │
                         ▼
┌──────────────────────────────────────────────────────┐
│ PREPROCESS UNDERSAMPLED MULTI-COIL K-SPACE             │──── S220
└──────────────────────────────────────────────────────┘
                         │
                         ▼
┌──────────────────────────────────────────────────────┐
│ RECONSTRUCT PREPROCESSED MULTI-COIL K-SPACE            │
│ BY USING MULTI-BAND JOINT CS RECONSTRUCTION            │──── S230
└──────────────────────────────────────────────────────┘
                         │
                         ▼
                    ┌─────────┐
                    │   END   │
                    └─────────┘
```

# FIG. 3B

```
ACQUIRE UNDERSAMPLED MULTI-COIL
K-SPACE DATA                              — S301

PERFORM INITIALIZATION OF DATA FOR FILLING
UNSAMPLED POSITIONS IN K-SPACE           — S302       — S309

PERFORM COIL COMPRESSION                 — S303

PERFORM MULTI-BAND DECOMPOSITION         — S304

OBTAIN COIL SENSITIVITY MAPS             — S305

PERFORM MULTI-DICTIONARY JOINT CS                     — S310
RECONSTRUCTION IN EACH FREQUENCY BAND    — S306

COMBINE RECONSTRUCTED MULTI-COIL K-SPACES
CORRESPONDING TO DIFFERENT FREQUENCY BANDS — S307

CALCULATE RESULTING MR IMAGE             — S308
```

# FIG. 4

```
                    ┌─────────┐
                    │  START  │
                    └─────────┘
                         │
                         ▼
        ┌──────────────────────────────────┐
        │  INITIALIZE AUXILIARY VARIABLES   │── S401
        └──────────────────────────────────┘
                         │
                         ▼
              YES     ╱ OUTER LOOP ╲   S402
        ┌────────────◀  PERFORMED?  ▶──────────────┐
        │            ╲             ╱                │
  S410  │                 │ NO                      │  S409
        ▼                 ▼                         ▼
┌─────────────────┐       ╱ INNER LOOP ╲   ┌─────────────────┐
│ RECONSTRUCT     │      ◀  PERFORMED?  ▶  │ UPDATE INNER    │
│ SAMPLES         │      ╲             ╱   │ LOOP            │
│ MEASURED IN     │  S403     │ NO     YES │ INDEPENDENT     │
│ RECONSTRUCTED   │           ▼        │   │ VARIABLES       │
│ MULTI-COIL      │                    │   └─────────────────┘
│ K-SPACE         │                    │            ▲
└─────────────────┘                    └────────────┘
        │
        ▼
   ┌─────────┐
   │   END   │
   └─────────┘
```

              ▼
┌──────────────────────────────────┐
│ COMBINE MULTI-COIL K-SPACES       │── S404
│ AND ESTIMATE OBJECT               │
└──────────────────────────────────┘
              │
              ▼
┌──────────────────────────────────┐
│ UPDATE ESTIMATION OF OBJECT       │── S405
│ BY MINIMIZING $\ell_2$ NORM       │
└──────────────────────────────────┘
              │
              ▼
┌──────────────────────────────────┐
│ UPDATE SPARSE OBJECT              │── S406
│ APPROXIMATION BY REDUCING $\ell_1$ NORM │
└──────────────────────────────────┘
              │
              ▼
┌──────────────────────────────────┐
│ UPDATE BREGMAN PARAMETERS         │── S407
└──────────────────────────────────┘
              │
              ▼
┌──────────────────────────────────┐
│ SPLIT UPDATED OBJECT AND          │── S408
│ RECALCULATE MULTI-COIL K-SPACE    │
└──────────────────────────────────┘

# FIG. 5

START

UNSAMPLED MULTI-COIL K-SPACE —S501

CONVERGED? —S502

RECONSTRUCTED MULTI-COIL K-SPACE —S519

RECONSTRUCT SAMPLES MEASURED IN MULTI-COIL K-SPACE —S518

OUTPUT RECONSTRUCTED MULTI-COIL K-SPACE —S503

INTERMEDIATE MULTI-COIL K-SPACE —S517

END

NO

YES

SPLIT INTO COIL IMAGES AND PERFORM FFT —S516

COIL SENSITIVITY MAPS —S505

S504— PERFORM IFFT AND MERGE IMAGES

INTERMEDIATE SPARSE APPROXIMATED MERGED OJBECT —S515

S506— MERGED IMAGE OF OBJECT

ASSEMBLIE SPARSE APPROXIMATED PATCHES —S514

S507— EXTRACT PATCHES

DICTIONARY —S510

S508— PATCHES

SPARSE APPROXIMATED PATCHES —S513

S512— APPROXIMATION OF PATCHES

S509— SPARSE OBJECT DECOMPOSITION

S511— SPARSE CODES

# FIG. 6

```
                                              ┌─────────────────────────┐
                                              │        PATCHES          │── S600
                                              └─────────────────────────┘
                                                          │
                                                          ▼
┌────────────────────────┐              ┌─────────────────────────┐
│       DICTIONARY        │── S602       │   SPARSE DECOMPOSITION   │── S601
└────────────────────────┘              └─────────────────────────┘
             │                                           │
             ▼                                           ▼
┌────────────────────────┐              ┌─────────────────────────┐
│  APPROXIMATE PATCHES    │◄─────────────│      SPARSE CODES       │── S603
└────────────────────────┘    S604      └─────────────────────────┘
             │
             ▼
┌────────────────────────┐
│     APPROXIMATIONS      │── S605
│      OF PATCHES         │
└────────────────────────┘
```

S602 — DICTIONARY

S601 — SPARSE DECOMPOSITION

S604 — APPROXIMATE PATCHES

S603 — SPARSE CODES

S605 — APPROXIMATIONS OF PATCHES

# FIG. 7

S700 — PATCHES

S701 — SPARSE DECOMPOSITION

S703 — SPARSE CODES FOR PATCHES

S702 — ORIGINAL-ORIENTED DICTIONARY

S704 — APPROXIMATE PATCHES

S705 — APPROXIMATIONS OF PATCHES

S706 — (−)

S707 — RESIDUALS OF PATCHES

S708 — SPARSE DECOMPOSITION

S710 — SPARSE CODES FOR RESIDUALS

S709 — RESIDUAL-ORIENTED DICTIONARY

S711 — APPROXIMATE RESIDUALS

S712 — APPROXIMATIONS OF RESIDUALS

S713 — (⊗)

S714 — MULTI-DICTIONARY APPROXIMATION OF PATCHES

# FIG. 8A

IMAGE      PATCH      DICTIONARY      SPARSE CODES

# FIG. 8B

# FIG. 8C

# FIG. 9

S900 — DATASET OF HIGH QUALITY IMAGES OF OBJECT → LEARN ORIGINAL-ORIENTED DICTIONARY — S901

S903 — CALCULATE RESIDUALS FOR IMAGES FROM DATASET ← ORIGINAL-ORIENTED DICTIONARY — S902

S904 — DATASET OF RESIDUALS → LEARN RESIDUAL-ORIENTED DICTIONARY — S905

RESIDUAL-ORIENTED DICTIONARY — S906

# FIG. 10

1000

DATA-SAMPLING UNIT

1002

DATA-ACQUISITION UNIT

1001

SENSITIVITY-ESTIMATING UNIT

1003

PREPROCESSING UNIT

1005

CS MRI RECONSTRUCTION UNIT

1004

SPARSE-APPROXIMATING UNIT

# FIG. 11

CURRENT ITERATION NUMBER

CONTROL UNIT — 1103

CONTROL UNIT

DICTIONARY INPUT UNIT — 1104

OUTPUT UNIT — 1106

TRIGGER UNIT — 1105

MERGING UNIT — 1107

K-SPACE INPUT UNIT — 1100

SENSITIVITY INPUT UNIT — 1101

MULTI-DICTIONARY APPROXIMATING UNIT — 1108

MEASUREMENT RESTORING UNIT — 1110

SPLITTING UNIT — 1109

DATA-SAMPLING UNIT — 1102

# FIG. 12

# FIG. 13

# FIG. 14

# FIG. 15

| MEDICAL DOCTOR (OPERATOR) (1510) | MR SCANNER (1520) | MR IMAGE RECONSTRUCTION SYSTEM (1530) |
|---|---|---|

S1511
**PLACE PATIENT INTO MR SCANNER**

S1512
**SELECT SCANNING MODE FOR ORGAN TO BE INVESTIGATED**

S1513
**SELECT UNDERSAMPLED MASK FOR DATA ACQUISITION**

S1514
**SELECT IMAGE RECONSTRUCTION PARAMETER**

S1515
**START SCANNING PROCESS**

S1516
**SCAN OBJECT AND TRANSMIT UNDERSAMPLED SPECTRAL DATA**

S1517
**RECONSTRUCT 3D IMAGE FROM UNDERSAMPLED DATA**

S1518
**INVESTIGATE RECONSTRUCTED IMAGE**

# FIG. 16

| MEDICAL DOCTOR (OPERATOR) (1610) | MR SCANNER (1620) | CLOUD (RECONSTRUCTION SERVICE) (1630) |
|---|---|---|

S1611
PLACE PATIENT INTO MR SCANNER

S1612
SELECT DESIRED ACCELERATION FACTOR

S1613
START SCANNINGS PROCESS

S1614
SCAN OBJECT AND TRANSMIT UNDERSAMPLED SPECTRAL DATA

S1615
TRANSMIT UNDERSAMPLED DATA TO CLOUD TO CLOUD PROVIDING RECONSTRUCTION SERVICE

S1616
RECONSTRUCT 3D IMAGE FROM UNDERSAMPLED DATA

S1617
RETRANSMIT RECONSTRUCTED IMAGE

S1618
INVESTIGATE RECONSTRUCTED IMAGE

# FIG. 17

| MEDICAL DOCTOR (OPERATOR) (1710) | LASER SCANNER (1720) | IMAGE RECONSTRUCTION SYSTEM (COMPUTER) (1730) |
|---|---|---|

S1711

POSITION OBJECT OF INTEREST IN FRONT OF SCANNER

S1712

SCAN OBJECT OF INTEREST

S1713

SCAN TEST-OBJECT

S1714

RECEIVE SCAN DATA AND USED TRANSFER FUNCTIONS TOGETHER WITH IMPERFECT FILLING FACTORS

S1715

MODIFY USED TRANSFER FUNCTION BASED ON SCAN DATA OF TEST-OBJECT

S1716

PHASE RETRIEVAL FOR OBJECT OF INTEREST

S1717

INVESTIGATE RECONSTRUCTED IMAGE

**FIG. 18**

EP 3 346 443 B1

**REFERENCES CITED IN THE DESCRIPTION**

**Patent documents cited in the description**

- US 8222900 B2
- US 20140218026 A1
- US 8427156 B2
- US 7884604 B2
- WO 2013067546 A1
- US 20060029279 A
- WO 2002031756 A1
- US 8699773 B2
- US 20100239143 A1

- US 8587307 B2
- US 20130099786 A1
- WO 2014075005 A1
- WO 2012144957 A1
- CN 103505207 A
- WO 2012123921 A
- WO 2006106508 A2
- US 20120177128 A

**Non-patent literature cited in the description**

- **R. OTAZO et al.** Combination of compressed sensing and parallel imaging for highly accelerated first-pass cardiac perfusion MRI. *Magn Reson Med.,* 2010, vol. 64, 767-776
- **T. ZHANG et al.** Coil compression for accelerated imaging with Cartesian sampling. *Magn Reson Med.,* 2013, vol. 69, 571-582
- **BHAVE.** *A variable splitting based algorithm for Fast Multi-Coil Blind Compressed Sensing MRI reconstruction*
- **BERMAN.** Dictionary Learning for Compressive T2 Mapping with Non-Cartesian Trajectories and Parallel Imaging
- **ZHU.** *Sparse Decomposition Learning Based Dynamic MRI Reconstruction*
- **CABALLERO.** Dictionary Learning and Time Sparsity for Dynamic MR Data Reconstruction
- **GONG.** MRI Reconstruction by Learning the Dictionary of Spatialfrequency-Bands Correlation: A novel algorithm integratable with PI and CS to further push acceleration

- **M. AHARON ; M. ELAD ; A. BRUCKSTEIN.** K-SVD: An Algorithm for Designing Overcomplete Dictionaries for Sparse Representation. *IEEE Trans. Image Process.,* 2006, vol. 54, 4311-4322
- **T. GOLDSTEIN ; S OSHER.** The Split Bregman method for l1-regularized problems. *SIAM J. on Imag. Sciences,* 2009, vol. 2, 323-343
- **Y. WANG.** A fast algorithm for image deblurring with total variation regularization. *CAAM Technical Report,* 2007
- **T. GOLDSTEIN ; S. OSHER.** The Split Bregman method for l1-regularized problems. *SIAM J. on Imag. Sciences,* 2009, vol. 2, 323-343
- **A. MIGUKIN et al.** Phase retrieval in 4f optical system: background compensation and sparse regularization of object with binary amplitude. *Appl. Opt.,* 2013, vol. 52, A269-A280